# EUROPÄISCHE PATENTANMELDUNG

(11) **EP 3 659 989 A1**
(43) Veröffentlichungstag der Anmeldung: **03.06.2020**
(21) Anmeldenummer: 18209278.3
(22) Anmeldetag: 29.11.2018
(51) Int. Cl.: C04B 35/111, A61C 13/00, A61K 6/00, B28B 1/00, C03C 4/00, C03C 10/00, C04B 35/44, C04B 35/443, C04B 35/486, C04B 35/488, C04B 35/626, C04B 35/636

(54) **SCHLICKER UND VERFAHREN ZUR HERSTELLUNG VON KERAMISCHEN UND GLASKERAMISCHEN 3D STRUKTUREN**

(71) Anmelder: Ivoclar Vivadent AG, 9494 Schaan (LI); IO Tech Group, Ltd., London W10 6AZ (GB)
(72) Erfinder: BONDERER, Lorenz Josef, 7320 Sargans (CH); LAUBERSHEIMER, Jürgen Rudolf, 9470 Buchs (CH); WACHTER, Wolfgang Josef, 9494 Schaan (LI); RITZBERGER, Christian, 9472 Grabs (CH); ZENOU, Michael, Hasmonaim 7312700 (IL)
(74) Vertreter: Uexküll & Stolberg

(57) **Zusammenfassung**

Schlicker zur Herstellung von Keramik- oder Glaskeramikformteilen durch ein LIFT-Verfahren, der (a) Keramik- und/oder Glaskeramikpartikel, (b) Bindemittel, (c) mindestens eine Energietransformationskomponente und (d) mindestens ein Dispergiermedium enthält, sowie ein LIFT-Verfahren zur Herstellung von Keramik- oder Glaskeramikformteilen unter Verwendung des Schlickers.

## Beschreibung

Die vorliegende Erfindung betrifft Schlicker und Verfahren für die Herstellung von Keramik- oder Glaskeramikformteilen, wie dentalen Inlays, Onlays, Veneers, Kronen, Brücken und Gerüsten.

Unter dem Begriff "Additive Manufacturing" (AM), werden generative Fertigungsverfahren zusammengefasst, bei denen aus computergestützten Konstruktionsdaten (CAD-Daten) 3-dimensionale Modelle oder Bauteile hergestellt werden (A. Gebhardt, Vision of Rapid Prototyping, Ber. DGK 83 (2006) 7-12). Dabei handelt es sich um Verfahren, wie z.B. Stereolithographie (SL), selektives Lasersintern (SLS), 3-D-Printing, Fused Deposition Modelling (FDM), Ink-Jet-Printing (IJP), 3D-Plotting, Multi-Jet Modelling (MJM), Solid Freeform Fabrication (SFF), Laminated Object Manufacturing (LOM), Laser Powder Forming (LPF) und Direct Ceramic Jet Printing (DCJP), mit denen sich Modelle, Bau- oder Formteile auch in Kleinserie kostengünstig herstellen lassen (A. Gebhardt, Generative Fertigungsverfahren, 3. Aufl., Carl Hanser Verlag, München 2007, 77ff.). Bei der Stereolithographie handelt es sich um AM-Verfahren (A. Beil, Fertigung von Mikro-Bauteilen mittels Stereolithographie, Düsseldorf 2002, VDI-Verlag 3 ff.), bei denen ein Formteil auf der Basis von CAD-Daten schichtweise aus einem flüssigen und härtbaren Monomerharz aufgebaut wird.

Stereolithographische Verfahren zur Herstellung von dentalen Formkörpern, wie Inlays, Kronen oder Brücken, sind vor allem bei keramischen Werkstoffen von Vorteil, weil damit eine deutliche Vereinfachung der im zahntechnischen Labor mit erheblichen manuellen Aufwand betriebenen Abform- und Gießprozesse bzw. der Fräs- und Schleifoperationen möglich ist und gleichzeitig der bei nicht-generativen Verfahren auftretende große Materialverlust vermieden werden kann. Da heutzutage eine vollständige digitale Prozesskette etabliert ist, lassen sich die klassischen Verfahrensschritte zur Herstellung von z.B. mehrgliedrigen Brückengerüsten (Ausrichten im Artikulator, Wachsmodulation, Einbetten und Guss) durch die Digitalisierung des Modells, virtuelle Konstruktion des dentalen Formkörpers und dessen generative stereolithographische Fertigung ersetzen.

Bei der stereolithographischen Herstellung von keramischen Formteilen wird zunächst ein keramischer Grünling durch schichtweise Strahlungshärtung eines fließfähigen keramischen Schlickers hergestellt, der dann nach der Entbinderung zu einem dichten keramischen Formkörper gesintert wird. Der Grünling wird auch Grünkörper genannt. Als Entbinderung wird das Austreiben des Bindemittels bezeichnet. Hierbei wird das verwendete Bindemittel üblicherweise durch Erhitzen des Grünlings auf eine Temperatur von ca. 80°C bis 600°C entfernt. Wesentlich ist, dass die Bildung von Rissen und Deformationen weitestgehend vermieden wird. Durch die Entbinderung wird aus dem Grünling der sogenannte Weißling. Beim Entbindern wird das Bindemittel durch thermische und thermo-chemische Prozesse zu flüchtigen Komponenten abgebaut.

Das Entbindern ist ein kritischer Schritt des Verfahrens. Hier ist die Gefahr groß, dass das Bauteil durch Gase, die bei der Zersetzung der organischen Matrix entstehen, und den durch diese Gase ausgeübten Druck beschädigt wird. Besonders groß ist die Gefahr, dass kleine Defekte zwischen den einzelnen Bauschichten beim Entbindern zu Rissen oder gar zur vollständigen Zerstörung des Bauteils führen. Dieses Risiko kann dadurch reduziert werden, dass die Entbinderungsdauer erhöht wird, was jedoch die Prozesszeit stark verlängert.

Die Sinterung des Weißlings erfolgt im Sinterofen beim Hochtemperaturbrand. Dabei kommt es zur Verdichtung und Verfestigung des fein verteilten Keramikpulvers durch Temperatureinwirkung unterhalb der Schmelztemperatur der Hauptkomponenten, wodurch das poröse Bauteil kleiner wird und dessen Festigkeit zunimmt.

Die US 5,496,682 offenbart lichthärtbare Zusammensetzungen für die Herstellung dreidimensionaler Körper durch Stereolithographie, die 40 bis 70 Vol.-% Keramik- oder Metallpartikel, 10 bis 35 Gew.-% Monomer, 1 bis 10 Gew.-% Photoinitiator, 1 bis 10 Gew.-% Dispergiermedium und vorzugsweise auch Lösungsmittel, Weichmacher und Kopplungsmittel enthalten.

Die US 6,117,612 beschreibt Harze für die stereolithographische Herstellung von gesinterten Keramik- oder Metallteilen. Die Harze haben eine Viskosität von weniger als 3000 mPa·s. Zur ihrer Herstellung werden Monomere mit geringer Viskosität eingesetzt, vorzugsweise in wässriger Lösung. Durch die Verwendung von Dispersionsmitteln soll ein hoher Feststoffgehalt bei geringer Viskosität erzielt werden.

Die DE 10 2005 058 116 A1 offenbart Suspensionen zur stereolithographischen Herstellung von keramischen Implantaten auf die in der US 6,117,612 beschriebene Weise, die keine Verdünnungsmittel wie Wasser oder organische Lösungsmittel enthalten, da diese durch lokales Verdampfen beim Energieeintrag die Viskosität erhöhen sollen. Die Viskosität der Suspension wird durch Variation der Konzentration eines Dispergators auf weniger als 20 Pa·s eingestellt. Als Dispergator werden Alkylammoniumsalze von Copolymeren mit sauren Gruppen eingesetzt, wobei diese auch auf die Partikel des keramischen Pulvers geschichtet werden können.

In der US 2005/0090575 A1 werden Methoden und Zusammensetzungen für die stereolithographische Herstellung von keramischen Bauteilen beschrieben. Es wird angegeben, dass mit den aus US 5,496,682 bekannten flüssigen Materialien hergestellte Formteile weich sind und daher einen zusätzlichen Härtungsschritt erfordern, um Verformungen beim Brennen zu vermeiden, während aus pastenförmigen Materialien gewonnene Formkörper bei der Entbinderung innere Spannungen aufbauen, die beim Sintern zu Rissen führen. Zur Vermeidung dieser Probleme werden Weichmacher eingesetzt und die Menge des Keramikpulvers so gewählt, dass die Viskosität der Zusammensetzungen mindestens 10.000 Pa·s beträgt.

Die EP 2 151 214 A1 offenbart lichthärtende Schlicker zur stereolithographischen Herstellung von Keramik- und Glaskeramikformteilen auf der Basis von radikalisch polymerisierbarem Bindemittel, Polymerisationsinitiator und Füllstoff. Als polyreaktives Bindemittel sind Polymerisations- und Polyadditionsharze bevorzugt, die aus einer Mischung von niedermolekularen oder oligomeren Monomeren mit einer oder mehreren polyreaktionsfähige Gruppen bestehen. Als Füllstoffe dienen oberflächenmodifizierte Keramik- und/oder Glaskeramikpartikel.

Bei der Stereolithographie müssen die zu verdruckenden Materialien bei der Verarbeitung fließfähig und photoreaktiv sein, um kurze Belichtungs- und Prozesszeiten zu ermöglichen. Beim 3D Inkjet Printing, muss die Viskosität der zu verdruckenden Schlicker deutlich unter 1 Pa·s liegen, wobei eine Viskosität von weniger als 0,1 Pa·s bevorzugt ist.

Die Anforderungen an die Viskosität schließen höherviskose Materialen und Suspensionen mit einem hohen Füllstoffgehalt aus. Nachteilig an keramischen Schlickern für den Einsatz in diesen Verfahren ist zudem, dass sie im flüssigen Zustand bei längerem Stehen zur Entmischung und Sedimentation neigen, d.h., dass sich die im Schlicker dispergierten Partikel vorzeitig absetzen. Andererseits sind Werkstoffe mit hohem Füllstoffgehalt und somit hoher Viskosität bevorzugt, weil sich ein hoher Füllgrad positiv auf die Entbinderung und Sinterung auswirkt und diesen Prozess stabilisiert und verkürzt. Zudem wird die Sedimentationsstabilität verbessert.

Aus der EP 2 233 449 A1 sind Schlicker zur Herstellung von Keramikformteilen durch Hot-Melt-Inkjet-Druckverfahren bekannt, die Keramikpartikel, Wachs und mindestens ein radikalisch polymerisierbares Wachs enthalten und die Grünkörper ergeben, die sich ohne Rissbildung entbindern lassen. Bei Hot-Melt-Inkjet-Verfahren liegen die Schlicker nur während des Druckvorgangs, d.h. für einen relativ kurzen Zeitraum, in flüssiger Form vor, wodurch die Gefahr der Entmischung reduziert wird.

Bei stereolithographischen Verfahren wird ein möglichst hoher Volumenanteil an keramischen Partikeln im Schlicker angestrebt. Die hierdurch bedingte hohe Viskosität ist aber im Hinblick auf die Verdruckbarkeit der Materialien nachteilig. Außerdem sollen Schlicker für stereolithographische Verfahren eine hohe Reaktivität aufweisen, um so kurze Belichtungs- und Prozesszeiten zu ermöglichen. Weiterhin sind eine gute Grünkörperfestigkeit und gute Formstabilität, hohe Genauigkeit und Präzision nach dem Entbindern, Sintern und abschießenden Reinigen der Körper zu gewährleisten.

Die EP 1 268 211 B1 offenbart ein Druckverfahren, bei dem durch einen fokussierten Laserstrahl lokal eine Volumen- oder Positionsänderung in dem zu verdruckenden Material induziert wird, so dass sich ein Farbtröpfchen von der im wesentlichen homogenen Farbschicht ablöst und auf den Bedruckstoff übertragen wird. Dieses Verfahren wird als Laser Induced Forward Transfer (LIFT) Verfahren bezeichnet. Das zu verdruckende Material wird von dem sogenannten Donor oder Trägersubstrat auf das Empfängersubstrat (Akzeptor) übertragen. Das Trägersubstrat besteht aus einem Träger, der mit einer dünnen Schicht des zu verdruckenden Materials beschichtet ist. Diese Materialschicht wird mit einem Laser punktförmig bestrahlt und dabei erweicht oder geschmolzen und teilweise verdampft. Bei transparenten Trägern kann der Laser von der Rückseite durch den Träger hindurch auf das zu verdruckende Material fokussiert werden. Ist der Träger nicht transparent, wird der Träger durch den Laser erhitzt und das Material indirekt erweicht oder geschmolzen. Alternativ kann der Laser von oben direkt auf das Material gerichtet werden. Das Empfängersubstrat (Bedruckstoff) ist in einem geringen, genau einzuhaltenden Abstand zum Trägersubstrat angeordnet. Durch die Laserenergie wird ein Teil des zu verdruckenden Materials schlagartig verdampft. Die sich bildende Dampfwolke reißt eine kleine Menge des erweichten oder geschmolzenen Materials mit und scheidet es auf dem Empfängersubstrat ab.

Um das zu verdruckende Material zu verdampfen, muss das Laserlicht absorbiert und in Wärme umgewandelt werden. Bei Druckfarben wird der Laserstrahl in der Regel durch Farbpigmente absorbiert, die in den Farben enthalten sind. Alternativ kann eine Absorptionsschicht vorgesehen sein, die das Laserlicht absorbiert und die Energie dann an das zu verdruckende Material überträgt. Eine solche Absorptionsschicht ist üblicherweise zwischen dem Träger und dem zu verdruckenden Material angeordnet. Absorptionsschichten sind nachteilig, weil häufig Teile dieser Schicht zusammen mit der Druckfarbe auf das Empfängersubstrat übertragen werden.

Die Herstellung von Keramik- oder (Glas-)Keramikformteilen durch LIFT-Verfahren ist nicht bekannt.

Die bekannten Schlicker und Verfahren sind hinsichtlich der oben genannten Anforderungen nicht optimal. Der Erfindung liegt daher die Aufgabe zugrunde, Verfahren und Schlicker zur Herstellung von Keramik- und Glaskeramikformteilen durch Additive Manufacturing (AM) bereitzustellen, die den obigen Anforderungen genügen und die die genannten Nachteile nicht aufweisen. Die Schlicker sollen sich insbesondere für LIFT-Verfahren eignen und im flüssigen Zustand während des Prozess sedimentationsstabil sein. Schlicker und Verfahren sollen Grünlinge mit hoher Gründichte und Festigkeit ergeben, die sich ohne Verformung, Riss- oder Spannungsbildung entbindern lassen. Die Grünlinge sollen beim Sintern zu hochfesten Keramiken führen, die für dentale Zwecke geeignet sind. Zudem sollen unterschiedliche Materialien gemeinsam zu einem Objekt verarbeitet werden können.

Erfindungsgemäß wird diese Aufgabe durch Schlicker gelöst, die
(a) Keramik- und/oder Glaskeramikpartikel,
(b) vorzugswese mindestens ein Bindemittel,
(c) mindestens eine Energietransformationskomponente und
(d) mindestens ein Dispergiermedium
enthalten.

Diese Aufgabe wird weiterhin durch ein additives Verfahren zur Herstellung dreidimensionaler Objekte gelöst, das vorzugsweise die folgenden Schritte umfasst:
(1) Flächiges Aufbringen des Schlickers auf ein3n Träger in definierter Schichtdicke, vorzugsweise in einer Schichtdicke von 3 - 300 µm, besonders bevorzugt 10 - 100 µm,
(2) Transfer eines Anteils des Schlickers vom Trägersubstrat (Donor) auf ein Empfängersubstrat (Akzeptor) durch den lokalen, ortsselektiven Eintrag eines Energieimpulses, vorzugsweise eines Laserimpulses,
(3) Verfestigen des Schlickers auf dem Empfängersubstrat, vorzugsweise durch Trocknen, Strahlenhärtung oder Ändern des Aggregatszustandes (z.B. durch Temperaturänderung),
(4) Wiederholen der Schritte (1)-(3), um einen Grünling zu erhalten,
(5) ggf. Entfernen des Stützmaterials aus dem Grünling und optionales Reinigen des Grünlings,
(6) optionales Nachvergüten des Grünlings durch weiteres Härten, vorzugsweise durch Trocknung, Strahlung, Wärme oder eine Kombination davon,
(7) Wärmebehandlung des Grünlings zum Entfernen der nichtkeramischen Inhaltsstoffe, insbesondere des Bindemittels, (Entbinderung), um einen Weißlings zu erhalten, und Sintern des Weißlings zu einem keramischen Objekt,
(8) optionale mechanische Bearbeitung des Objekts, z.B. durch Trowalisieren oder manuelles Bearbeiten wie Schleifen und/oder Polieren.

Die Schritte (1) bis (3) werden so oft wiederholt, bis der gewünschte Grünkörper fertiggestellt ist. Gemäß einer bevorzugten Ausführungsform wird der Schlicker im Anschluss an Schritt (3) geglättet, vorzugsweise mit einer Walze, Klinge, Fräse und/oder einem Abstreifer. Anschließend wird der Grünkörper in den Schritten (5) bis (8) weiterverarbeitet, wobei die Schritte (5), (6) und (8) optional sind.

In dem Verfahren können ein oder mehrere unterschiedliche Schlicker eingesetzt werden.

Der oder die Schlicker können zusammen mit einem Stützmaterials verarbeitete werden. Das Stützmaterial wird aus dem fertigen Objekt entfernt. Dies kann entweder in einem separaten Verfahrensschritt (5) oder während des Entbinderns oder Sinterns in Schritt (7) erfolgen. Schlicker und Stützmaterial werden hierin auch als Druckmaterialen bezeichnet.

In Schritt (6) werden ggf. vorhandene ungehärtete Anteile des Bindemittels gehärtet, vorzugsweise durch Bestrahlung mit Licht, besonders bevorzugt mit sichtbarem oder UV-Licht. Diese Form der Nachbehandlung kommt insbesondere dann in Betracht, wenn das Bindemittel in Schritt (3) durch Bestrahlung mit Licht, vorzugsweise mit sichtbarem oder UV-Licht, gehärtete wird.

Bevorzugte Träger in **Schritt (1)** sind Polymerfolien, bevorzugt mit einer Dicke von 10 - 200 µm, insbesondere PET-, Polyimid-, und Polyvinylchlorid (PVC)-folien; Glasträger, vorzugsweise aus Floatglas oder Borosilikatglas; Träger aus nicht-metallischen, anorganischen, porösen oder nicht-porösen Werkstoffen; metallische Träger, vorzugsweise aus Edelstahl, Aluminium, Titanlegierungen, Kupferlegierungen wie Bronze oder Messing; Träger aus nicht-metallischen, anorganischen Werkstoffen wie keramische Träger, vorzugsweise aus ZrO₂, Al₂O₃, Zirconia toughened Alumina (ZTA), Alumina toughened Zirconia (ATZ), SiCₓ, SiNₓ, Diamond like carbon, Glassy Carbon, BN, B₄C oder AlN; oder Träger aus einer Kombinationen dieser Materialien. Die Träger werden so gewählt, dass sie sich gegenüber dem Schlicker ausreichend inert verhalten, d.h. insbesondere nicht innerhalb der Anwendungszeit merklich vom Dispergiermedium gequollen oder angegriffen werden.

Der Träger kann als Platte, Einwegband, Endlosband, Zylinder oder Hohlzylinder vorliegen. Die Arbeitsfläche kann eben oder gewölbt sein. Gewölbte Flächen sind bevorzugt um eine Achse gewölbt, wie z.B. die Mantelfläche eines Zylinders.

Um die Bildung homogener Schichten des Schlickers und ggf. des Stützmaterials zu unterstützen, werden Schlicker, Stützmaterial und Träger vorzugsweise aufeinander abgestimmt. Es wird eine geringe Grenzflächenspannung zwischen Schlicker bzw. Stützmaterial und Träger angestrebt. Für hydrophile Schlicker und Stützmaterialen werden vorzugsweise hydrophile Träger- und/oder Empfängersubstrate verwendet, beispielsweise Glasträger, Cellophan oder hydrophile PET-Folien.

Oberflächen können z.B. durch Flamm-, Plasma- oder Ätzbehandlungen hydrophilisiert werden. Generell sollen Schlicker und Stützmaterial den Träger gut benetzten. Durch die Zugabe eines Tensids zu Schlicker und Stützmaterial kann die Benetzung ebenfalls verbessert werden. Bei hydrophoben Schlickern und Stützmaterialen sind hydrophobe Träger bevorzugt.

Der Schlicker kann auf bekannte Weise auf den Träger aufgebracht werden, vorzugsweise durch Rakel- oder Doktorbladesysteme, mit Schlitzdüsen (mit oder ohne Dispenser), durch Flexo- oder Gravurdruck, Siebdruck, Tampondruck, Sprühbeschichtung oder durch eine Kombination dieser Verfahren. Generell sind hierzu alle im Stand der Technik bekannten Druckmethoden geeignet. Der beschichtete Träger wird hierin auch als Trägersubstrat bezeichnet.

Im Fall von Druckzylindern wird der Schlicker und ggf. das Stützmaterial vorzugweise kontinuierlich auf einen rotierenden Zylinder aufgetragen. Durch die Rotation wird die auf dem Zylinder gebildete Schicht des Materials in Richtung der Energiequelle, z.B. des Lasers, transportiert und dort verdruckt. Anschließend wird das verdruckte Material bei weiterer Rotation wieder ergänzt.

Trägerfolien können ebenfalls in kontinuierlichen Verfahren eingesetzt werden, beispielsweise indem sie als umlaufendes Band ausgebildet werden. Die beschichteten Folien können aber auch für die einmalige Verwendung konfektioniert sein.

In **Schritt (2)** wird ein Teil der eingebrachten Energie durch den Schlicker absorbiert und in Wärme umgewandelt. Die Absorption findet vorzugsweise ohne eine zusätzliche Absorptionsschicht auf dem Trägersubstrat im Schlicker selbst statt, so dass die mit derartigen Absorptionsschichten verbundenen Nachteile vermieden werden.

Die Energieabsorption bewirkt eine lokale, schlagartige Volumenausdehnung, beispielsweise durch Verdampfung, des Dispergiermediums im Material und führt zum Ablösen des Schlickers vom Trägersubstrat und zum Transfer auf das Empfängersubstrat. Dabei werden Tropfen des Schlickers auf das Empfängersubstrat übertragen, wo sie koaleszieren können und beispielsweise einen homogenen Film bilden.

Der Energieeintrag in Schritt (2) erfolgt vorzugsweise über die dem Schlicker abgewandte Seite des Trägersubstrats.

Das Empfängersubstrat kann eine plane Oberfläche aufweisen und sollte mindestens so groß sein, dass es das zu druckende Bauteil in Gänze aufnehmen kann. Das Empfängersubstrat hat vorzugsweise eine glatte Oberfläche, die geschlossen oder porös sein kann. Unter einer porösen Oberfläche wird eine Oberfläche verstanden, die Poren mit einer mittleren Größe von vorzugsweise 1 nm - bis 10 µm aufweist. Die Porengröße wird rasterelektronenmikroskopisch durch Auszählen bestimmt. Angegeben sind die dabei erhaltenen Mittelwerte.

Beispielhafte Materialien mit mikro- oder nanoporöser Oberfläche sind abgebundener, trockener Gips, angesintertes aber noch poröses ZrO₂, nanoporöses Glas oder mikroporöse Kunststoffe, wie z.B. zusammengesintertes Polyethylen hoher Dichte.

Durch die Verwendung von porösen Empfängersubstraten kann das Trocknen der Druckmaterialien begünstigt werden, besonders von solchen Druckmaterialien, die feste Partikel enthalten, wie Schlicker für die Herstellung von keramischen Objekten. Besonders dann, wenn das Verfestigen durch Trocknen stattfindet, kann ein separater Trocknungsschritt entfallen. Es ist jedoch darauf zu achten, dass die Poren kleiner als die festen Füllstoffpartikel sind, damit diese nicht beim Trocknen die Poren verstopfen.

Gemeinsam mit dem oder den Schlickern können ein oder mehrere Stützmaterialien verdruckt werden. Unter Stützmaterialien werden Materialen verstanden, die von dem fertigen Objekt entfernt werden. Die Schlicker bleiben nach dem Entfernen des Stützmaterials zurück und bilden das gewünschte Objekt.

Bevorzugt sind schmelzbare, rückstandsfrei verbrennende Stützmaterialien, die beim Entbindern schmelzen und entfernt werden und/oder die beim Sintern verbrannt werden. Alternativ kann das Stützmaterial auch vor dem Entbinderungs- und SinterProzess z.B. durch Lösen, Schmelzen, Abwaschen oder einer Kombination davon entfernt werden.

Die gewünschten dreidimensionalen Objekte werden durch wiederholtes schichtweises Verdrucken von Schlicker und ggf. Stützmaterial hergestellt. Die einzelnen Schichten können allein durch das Stützmaterial, allein durch den Schlicker oder durch beide Materialien gemeinsam gebildet werden. Durchgehende Schichten, die ausschließlich durch das Stützmaterial gebildete werden, sind naturgemäß so angeordnet, dass sie außerhalb des fertigen Objekts liegen, d.h. beispielsweis oberhalb oder unterhalb des Objekts.

Schlicker und Stützmaterial können in einem Arbeitsgang gemeinsam oder nacheinander verdruckt werden. Beispielswese kann in einem ersten Arbeitsgang ein Stützmaterial verdruckt und dann in oder auf die verfestigte Stützstruktur auf die beschriebene Weise der Schlicker gedruckt werden. Dabei können die abgeschiedenen Schichtstärken des Stützmaterials und des Schlickers unterschiedlich sein. Dadurch kann es z.B. notwendig werden, dass die Anzahl abgeschiedener Schichten für Schlicker und Stützmaterial unterschiedlich sind. Gemäß einer bevorzugten Ausführungsform werden zuerst mehrere Schichten des Stützmaterials auf dem Empfängersubstrat abgeschieden.

Anschließend wird das gewünschte Objekt durch Verdrucken von mindestens einem Schlicker gebildet. Nach der Fertigstellung des eigentlichen Bauteils können weitere Schichten des Stützmaterials aufgebracht werden, so das Ober- und Unterseite des gedruckten Objekts durch ein oder mehrere Schichten des Stützmaterials begrenzt sind. In einer besonders bevorzugten Ausführungsform wird in jeder Schicht der äußere Rand um das Bauobjekt herum durch das Stützmaterial gebildet, so dass das gedruckte Objekt allseitig von Stützmaterial umschlossen ist. In Bereichen des Bauteils, in denen sich der Querschnitt nicht stark verändert, können dickere Schichten verwendet werden, während bei Stellen, in denen sich der Bauteilquerschnitt schnell verändert, dünnere schichten bevorzugt sind.

Optional kann die aufgebrachte Materialschicht zur Vorbereitung für den nächsten Auftragungszyklus in einem weiteren Verfahrensschritt geglättet werden, beispielsweise mit einer Metallwalze, einer Klinge, einem Abstreifer oder einer Fräse mit/ohne Materialabsaugung.

Das schichtweise Aufbringen wird so lange fortgesetzt, bis das gewünschte dreidimensionale Objekt fertiggestellt ist. Der Druckvorgang wird wie bei additiven Fertigungsverfahren üblich durch einen Computer mittel CAD-Daten gesteuert. Dabei wird der Schlicker in den Bereichen verwendet, die das Formteil bilden, und das Stützmaterial unterhalb von Überhängen, seitlich des Bauteils und in Hohlräumen.

In einer bevorzugten Ausführungsform des Verfahrens wird das Druckmaterial, d.h. Schlicker oder Stützmaterial, während des Druckprozesses auf den Träger aufgebracht. Alternativ können auch bereits vorgängig beschichtete Substrate verwendet werden, bevorzugt in Form von beschichteten Trägerfolien. Vorzugsweise wird durch erneutes, selektives oder kontinuierliches Beschichten des Trägersubstrats neues Druckmaterial für den LIFT-Vorgang zur Verfügung gestellt.

Das erfindungsgemäße Verfahren ist vorzugsweise ein LIFT-Verfahren. Unter einem LIFT-Verfahren wird hier ein Verfahren verstanden, bei dem wie eingangs erläutert durch einen Energieimpuls aus einem Druckmaterial eine kleine Materialmenge herausgelöst und auf ein Empfängersubstrat übertragen wird. Der Energieimpuls wird vorzugsweise durch einen Laser erzeugt. Der Laserstrahl wird dabei auf einen kleinen Bereich des Schlickers oder Stützmaterials fokussiert und hierdurch das Druckmaterial lokal so stark erhitzt, dass sich zumindest ein Bestandteil des Schlickers schlagartig ausdehnt. Dieser Bestandteil wird auch als Volumenausdehnungskomponente bezeichnet und entspricht erfindungsgemäß dem Dispergiermedium. Die Energietransformationskomponente absorbiert die Laserenergie und überträgt diese auf den Schlicker oder das Stützmaterial. Die schlagartig verdampfende Volumenausdehnungskomponente reißt den Schlicker oder das Stützmaterial mit und überträgt es auf das Empfängersubstrat. Es ist auch möglich, dass die Volumenausdehnungskomponente einen Teil der Energie direkt absorbiert.

Erfindungsgemäß kann anstelle eines Laserstrahls eine andere geeignete Energiequelle verwendet werden, beispielsweise fokussiertes Licht (nicht Laser) oder Teilchenstrahlen wie Elektronen- oder Ionenstrahlen. Der Einfachheit halber wird hier der Begriff LIFT-Verfahren auch für Verfahren verwendet, in denen kein Laser eingesetzt wird. Bevorzugt sind Laser, insbesondere Laser mit einer Wellenlänge von 300 nm bis 4000 nm, beispielsweise Neodym-YAG Laser mit einer Wellenläge von 1064 nm. Besonders bevorzugt ist gepulstes Laser-Licht mit einer Pulsenergie im µJ Bereich und einer Pulsdauer von 1 ns bis 1 µs.

Die erfindungsgemäß in dem LIFT-Verfahren eingesetzten Schlicker enthalten vorzugsweise die folgenden Bestandteile.

Als **Keramik- und/oder Glaskeramikpartikel (a)** eignen sich Keramik- und/oder Glaskeramikpulver, die nach einem thermischen Entbinderungs- und Sinterprozess keramische Körper mit einer gewünschten Festigkeit ergeben, beispielsweise Oxid- oder Glaskeramikpartikel, wie z.B. Aluminiumoxid-, Zirkonoxid- oder Lithiumdisilikatpartikel. Erfindungsgemäß sind Partikel mit einer Partikelgröße von weniger als 20 µm bevorzugt, besonders bevorzugt von weniger als 10 µm und ganz besonders bevorzugt von weniger als 5 µm. Die Partikel liegen vorzugsweise in nicht agglomerierter Form vor. Besonders bevorzugt sind Partikel mit einer Partikelgröße von 3 nm bis 20 µm, ganz besonders bevorzugt von 5 nm bis 10 µm und insbesondere von 7 nm bis 5 µm.

Wenn nicht anders angegeben handelt es sich bei allen Partikelgrößen hierin um den Mittelwert (d₅₀-Wert) der Volumenverteilung, die durch Dynamic Light Scattering für Partikel kleiner als 5 Mikrometer und durch Statische Lichtstreuung für Partikel größer als 5 Mikrometer gemessen wird. Zur Messung der Partikelgröße werden die zu Partikel in einer Konzentration von 0,1 Gew.-% in einer geeigneten Flüssigkeit suspendiert. Wenn die Partikel hydrolysebeständig sind (z.B. ZrO₂, Al₂O₃, ZTA, ATZ), wird deionisiertes Wasser verwendet. Der pH wird mit einer Säure oder Base so eingestellt, dass er mindestens 2, besser 3 pH-Einheiten vom isoelektrischen Punkt (Literaturwerte) der Partikel entfernt ist. Die Proben werden vor und während der Messung mit Ultraschall behandelt. Bei hydrolyseempfindlichen Partikeln (z.B. Lithiumdisikilat) wird ein Lösungsmittel verwendet, das die Partikel nicht angreift, beispielsweise Tripropylenglycol. In diesem Fall wird der pH nicht angepasst. Zur Verbesserung der Dispergierbarkeit kann ein geeignetes Oberflächenmodifizierungsmittel zugesetzt werden, beispielsweis Lubrizol™ Solplus D540.

Die Bestimmung der Teilchengröße erfolgt insbesondere mit dem Verfahren der statischen Laserbeugung (SLS) nach ISO 13320:2009, z.B. unter Benutzung eines Partikelanalysators LA-960 der Firma Horiba, oder der dynamischen Lichtstreuung (DLS) nach ISO 22412:2017, z.B. unter Benutzung eines Partikelmessgeräts Nano-flex der Firma Colloid Metrix.

Unter Keramiken werden anorganische Werkstoffe verstanden, die eine kristalline Struktur aufweisen und meist aus entsprechenden Pulvern hergestellt werden. Vorzugsweise erfolgt die Herstellung der Keramik durch Sintern (Sinterkeramik). Oxidkeramiken werden vorzugsweise durch Sintern von Metalloxidpulvern wie z.B. ZrO₂ oder Al₂O₃ erhalten. Darüber hinaus können Oxidkeramiken auch eine oder mehrere Kristallphasen enthalten.

Bevorzugt sind Keramikpartikel auf der Basis von ZrO₂ oder Al₂O₃, Partikel auf der Basis von mit CaO, Y₂O₃, La₂O₃, CeO₂ und/oder MgO stabilisiertem ZrO₂, Partikel auf der Basis von anderen Metalloxiden oder von Keramiken, die aus mehreren Oxiden hergestellt werden und somit aus unterschiedlichen kristallinen Oxidphasen aufgebaut sind, vorzugsweise ZrO₂-Al₂O₃, ZrO₂-Spinell, ZrO₂-Al₂O₃-Spinell, Spinelle des Typs AB₂O₄, AB₁₂O₁₉, AB₁₁O₁₈, AB₂O₄ verwendet werden, wobei A bevorzugt ein Alkali- oder Erdalkalimetallion und B ein Übergangsmetallion ist, das eine höhere Oxidationsstufe als A aufweist. Ganz besonders bevorzugt sind Keramikpartikel aus ZrO₂-Al₂O₃, ZrO₂-Spinell, ZrO₂-Al₂O₃-Spinell oder aus mit CaO, Y₂O₃, La₂O₃, CeO₂ und/oder MgO stabilisiertem ZrO₂-Al₂O₃.

Stabilisierte Keramiken enthalten neben dem Basisoxid wie ZrO₂ oder Al₂O₃ ein Stabilisierungsmittel, das vorzugsweise aus CaO, Y₂O₃, La₂O₃, CeO₂, MgO, Er₂O₃ und Mischungen davon ausgewählt ist. Das Stabilisierungsmittel wird vorzugsweise in einer Menge von 2 bis 14 mol.-% eingesetzt, bezogen auf die Masse der stabilisierten Keramik. Hochfeste ZrO₂-Keramiken enthalten zur Stabilisierung der tetragonalen und/oder kubischen Kristallstruktur vorzugsweise 2 bis 12 mol.-% Y₂O₃ (Yttriumoxid), besonders bevorzugt 2 bis 10 mol.-%. Diese ZrO₂-Keramik wird als Y-TZP (Yttrium Stabilized Tetragonal Zirconium Dioxide Polycrystals) bezeichnet. Keramikpartikel, die nur Basisoxid und Stabilisierungsmittel enthalten, sind besonders bevorzugt.

Im finalen Endprodukt nach dem Dichtsintern erhält man bei höheren Anteilen an Y₂O₃ (>3 mol.-%) meist ein Kristallphasengemisch, d.h. eine Mischung aus tetragonaler und kubischer Kristallphase. Tetragonales stabilisiertes Zirkonoxid wird als "Partial stabilized zirconia" (PSZ) bezeichnet. Kubisch stabilisiertes Zirkonoxid wird als "Fully stabilized zirconia" bezeichnet. Bei Anteilen von 2 bis 3 mol.-% ZrO₂ ist der Hauptkristallphasenanteil meist tetragonal. Mit steigendem Y₂O₃-Anteil nimmt der Anteil der kubischen Kristallphase jedoch zu. Erfindungsgemäß sind weiterhin Schlicker bevorzugt, die als Komponente (a) Glaskeramikpartikel enthalten. Bei Glaskeramiken handelt es sich um Werkstoffe, die meist aus amorphen Gläsern, insbesondere Silikatgläsern, durch gesteuerte Keimbildung und Kristallisation hergestellt werden und bei denen eine Glasphase und eine oder mehrere Kristallphasen im Festkörper nebeneinander vorliegen. Bei Glaskeramiken kann sowohl von Glaspulvern als auch von Glaskeramikpulvern ausgegangen werden. Besonders bevorzugt sind Glaskeramikpartikel, die Leucit- und ganz besonders bevorzugt Lithiumdisilikatkristalle enthalten. Diese lassen sich vorteilhaft aus Lithiumdisilikat-Glaspulver oder Lithiumdisilikat-Glaspulvern durch thermische Behandlung (Kristallisations- und Sinterbrand) herstellen. Erfindungsgemäß bevorzugte Glaskeramiken werden in EP 1 505 041 A1, EP 2 261 184 A1, EP 2 377 830 A1 und EP 2 377 831 A1 im Einzelnen beschrieben.

Die optimale Partikelgröße der Komponente (a) ist abhängig von der eingesetzten Keramik. Bei Al₂O₃ liegt die Größe der als Komponente (a) verwendeten Partikel vorzugsweise im Bereich von 50 bis 500 nm, besonders bevorzugt zwischen 75 und 200 nm; bei Glaskeramik im Bereich von 200 nm bis 20 µm, besonders bevorzugt im Bereich von 500 nm bis 10 µm, und ganz besonders bevorzugt im Bereich von 1 und 5 µm; bei TZP-3Y Zirkondioxid im Bereich von 3 bis 500 nm, ganz bevorzugt im Bereich von 20 und 350 nm. Die Partikelgröße wird dabei vorzugsweise so gewählt, dass sedimentationsstabile Schlicker erhalten werden.

Weiterhin lassen sich auch Keramik- oder Glaskeramikpartikel mit einer Partikelgröße im Bereich von 10-200 nm als Nano- der Organosole, d.h. als Dispersion der Nanopartikel in einem geeigneten Dispergiermedium einsetzen.

Die als Komponente (a) eingesetzten Keramik- oder Glaskeramikpartikel sind vorzugsweise eingefärbt. Hierzu werden die Keramik- und/oder Glaskeramikpartikel vorzugsweise mit einem oder mehreren **Farbmitteln (e)** gemischt.

Das Farbmittel (e) wird vorzugsweise in einer Menge von 0,00001 bis 10 Gew.-%, besonders bevorzugt 0,0001 bis 7 Gew.-% und ganz besonders bevorzugt 0,001 bis 5 Gew.-% eingesetzt, bezogen auf die Masse der Komponente (a).

Als Farbmittel sind die üblichen Farbstoffe oder Pigmente erfindungsgemäß nicht geeignet, da sie nicht genügend stabil sind, um den Entbinderungs- oder Sinterungsprozess zu überstehen. Erfindungsgemäß werden als Komponente (e) vorzugsweise reaktive Übergangsmetallverbindungen eingesetzt, die während der Entbinderung des hergestellten Keramikgrünlings oder der Sinterung des daraus erhaltenen Keramikweißlings farbgebende Übergangsmetallionen bilden. Als Farbmittel bevorzugte Übergangsmetallverbindungen sind vor allem Acetylacetonate oder Carbonsäuresalze der Elemente Eisen, Cer, Praseodym, Terbium, Lanthan, Wolfram, Osmium, Terbium und Mangan. Bevorzugt sind weiterhin die Salze der Carbonsäuren Essig-, Propion-, Butter-, 2-Ethylhexylcarbon-, Stearin- und Palmitinsäure. Besonders bevorzugt sind vor allem die entsprechenden Fe-, Pr-, Mn- und Tb-Verbindungen, wie z.B. Eisen(III)-acetat oder -acetylacetonat, Mangan(III)-acetat oder -acetylacetonat, Praseodym(III)-acetat oder -acetylacetonat oder Terbium(III)-acetat oder -acetylacetonat sowie die entsprechenden Carbonsäuresalze.

Vorzugsweise werden die farbgebenden Komponenten so gewählt, dass nach dem Entbindern und Sintern zahnfarbene Keramikformteile erhalten werden.

Weitere bevorzugte Farbmittel (e) sind Pigmente, besonders bevorzugt anorganische Pigmente, wie beispielsweise die Oxide von Pr, Tb, Ce, Co, Ni, Cu, Bi, La, Nd, Sm, Eu, Gd und insbesondere von Fe, Mn, Cr, Er. Zur Einfärbung der Keramik- oder Glaskeramikpulver können vorteilhaft auch farbige dotierte Spinell- oder ZrO₂-Verbindungen des Typs AB₂O₄ oder ZrO₂:X verwendet werden, wobei A bevorzugt ein Alkali- oder Erdalkalimetallion und B oder X ein Übergangsmetallion ist, das eine höhere Oxidationsstufe als A hat. Die dotierten Spinelle und dotierten ZrO₂-Pigmente eignen sich besonders zur Einfärbung von Glaskeramik und insbesondere von Lithiumdisilikatglaskeramik. Die farbgebenden Komponenten werden in einer Menge zugesetzt, die erforderlich ist, um die gewünschte Färbung zu erzielen. Vorzugsweise werden Art und Menge der farbgebenden Komponente(n) so gewählt, dass nach dem Entbindern und Sintern zahnfarbene Keramikformteile erhalten werden. Der Farbeindruck wird erst während des Sinterns endgültig ausgebildet.

Gemäß einer alternativen Ausführungsform sind die farbgebenden Komponenten bereits in den Keramik- oder Glaskeramikpartikel enthalten. Ein Beispiel hierfür sind ZrO₂-Partikel mit 3 mol.-% Er₂O₃, ZrO₂-Partikel mit 3 mol.-% Y₂O₃ und Fe₂O₃ (gelb), ZrO₂-Partikel mit 3 mol.-% Y₂O₃ und Co₂O₃ (grau), ZrO₂-Partikel mit 5 mol.-% Y₂O₃ und Fe₂O₃ (gelb) und ZrO₂-Partikel mit 5 mol.-% Y₂O₃ und Co₂O₃ (grau)

Erfindungsgemäß geeignete, gefärbte Keramik- oder Glaskeramikpulver sind zum Teil kommerziell erhältlich.

Rot eingefärbte Partikel auf der Basis von mit Er₂O₃ stabilisiertem ZrO₂ sind von der Firma Tosoh unter der Bezeichnung TZ-PX-389 erhältlich. Diese Partikel enthalten kein Y₂O₃. Die Farbwirkung ist primär auf Er₂O₃ zurückzuführen. Der Anteil von Er₂O₃ ist 9,2 Gew.-% (bzw. 5 mol.-%).

Partikel auf der Basis von mit 3 mol.-% Y₂O₃ stabilisiertem ZrO₂, die 0,15 Gew.-% Fe₂O₃ enthalten, weisen eine gelbe Farbe und eine niedrige Transluzenz auf. Derartige Partikel sind von der Firma Tosoh unter der Bezeichnung TZ-PX364 erhältlich.

Partikel auf der Basis von mit 5 mol.-% Y₂O₃ stabilisiertem ZrO₂, die 0,25 Gew.-% Fe₂O₃ enthalten, weisen eine gelbe Farbe und eine hohe Transluzenz auf. Derartige Partikel sind von der Firma Tosoh unter der Bezeichnung TZ-PX590 erhältlich.

Partikel auf der Basis von mit 3 mol.-% Y₂O₃ stabilisiertem ZrO₂, die 0,04 Gew.-% Co₂O₃ enthalten, weisen eine graue Farbe und eine niedrige Transluzenz auf.

Partikel auf der Basis von mit 5 mol.-% Y₂O₃ stabilisiertem ZrO₂, die 0,04 Gew.-% Co₂O₃ enthalten, weisen eine graue Farbe und eine hohe Transluzenz auf.

Die Transluzenz von Keramikpartikeln auf der Basis von ZrO₂ kann durch den Gehalt an Y₂O₃ eingestellt werden. Kommerziell erhältlich sich Keramiken mit 3 mol.-% (Tosoh TZ-PX-245), 4 mol.-% (Tosoh TZ-PX-524), 4,25 mol.-% (Tosoh TZ-PX-551) und 5 mol.-% (Tosoh TZ-PX-430) Y₂O₃, wobei die Transluzenz mit steigendem Y₂O₃-Gehalt zunimmt.

Zur Erzielung des gewünschten Farbeindrucks ist es auch möglich, Mischungen von unterschiedlich eingefärbten Keramik- oder Glaskeramikpulvern als Komponente (a) einzusetzen. Hierzu werden unterschiedlich gefärbte Keramik- und/oder Glaskeramikpartikel bzw. Farbmittel so miteinander gemischt, dass ein Schlicker mit der gewünschten Farbe erhalten wird, und dieser Schlicker wird anschließend verdruckt.

Erfindungsgemäß können zur Herstellung von Keramik- oder Glaskeramikformteilen auch mehrere unterschiedlich gefärbte Schlicker einsetzt werden, die erst beim LIFT-Prozess so miteinander kombinieren werden, dass ein Körper mit der gewünschten Färbung erhalten wird. Diese Vorgehensweise hat den Vorteil, dass unterschiedliche Färbungen innerhalb des Körpers erhalten werden können.

Gemäß einer bevorzugen Ausführungsform der Erfindung werden die Partikel der Komponente (a) mit geeigneten Stoffen oberflächenmodifiziert. Zur Oberflächenmodifizierung werden vorzugsweise solche Verbindungen eingesetzt, die chemisch, d.h. durch ionische oder kovalente Bindungen, an die Oberfläche der Keramik- oder Glaskeramikpartikel gebunden werden. Bevorzugt sind Verbindungen, die entweder Säuregruppen, vorzugsweise Silylgruppen, vorzugsweise Alkoxysilylgruppen aufweisen. Die Partikeloberfläche kann teilweise oder vorzugsweise vollständig mit dem Modifizierungsmittel bedeckt sein. Bei den erfindungsgemäß verwendeten Modifizierungsmitteln handelt es sich vorzugsweise um oligomere oder polymere Verbindungen.

Erfindungsgemäß sind solche Verbindungen besonders geeignet, die im Gegensatz zu den sogenannten Haftvermittlern oder Kopplungsreagenzien nur mit der Partikeloberfläche reagierende Gruppen enthalten, aber keine radikalisch polymerisierbaren Gruppen, die mit der Harzmatrix eine kovalente Bindung eingehen. Solche Verbindungen werden hierin als nicht polymerisierbare Oberflächenmodifizierungsmittel bezeichnet. Diese Verbindungen haben den Vorteil, dass kein stabiler Verbund zwischen der Keramikpartikeloberfläche und der Polymermatrix im ausgehärteten Grünling zustande kommt, was die vollständige Entfernung der Polymeranteile im Entbinderungsprozess vereinfacht.

Als nicht polymerisierbare Oberflächenmodifizierungsmittel eigen sich insbesondere Silane, ganz besonders Propyltrimethoxysilan, Phenyltrimethoxysilan, Hexyltrimethoxysilan, Octyltrimethoxysilan, Trimethylchlorsilan, Trimethylbromsilan, Trimethylmethoxysilan oder Hexamethyldisilazan. Oberflächenmodifizierungsmittel werden ggf. in einer Menge von vorzugsweise 0,01 bis 5 Gew.-%, besonders bevorzugt 0,1 bis 2 Gew.-% und ganz besonders bevorzugt 0,2 bis 1,5 Gew.-% bezogen auf die Masse des Schlickers eingesetzt.

Die erfindungsgemäßen Schlicker enthalten vorzugsweise ein **Bindemittel (b).** Die Schlicker können als Bindemittel (b) z.B. ein oder mehrere radikalisch polymerisierbare Monomere enthalten, vorzugsweise mindestens ein (Meth)acrylat und/oder (Meth)acrylamid, vorzugsweise mono- oder multifunktionelle (Meth)acrylate oder deren Mischung. Besonders bevorzugt sind Werkstoffe, die als radikalisch polymerisierbares Monomer mindestens ein multifunktionelles (Meth)acrylat oder eine Mischung von mono- und multifunktionellen (Meth)acrylaten enthalten. Unter monofunktionellen (Meth)acrylaten werden Verbindungen mit einer, unter polyfunktionellen (Meth)acrylaten Verbindungen mit zwei oder mehr, vorzugsweise 2 bis 6 radikalisch polymerisierbaren Gruppen verstanden. Bevorzugte Bindemittel dieses Typs werden in der EP 3 147 707 A1 in den Absätzen [0036] bis [0044] beschrieben. Daraus geht auch hervor, dass derartige Bindemittel vorzugweise einen Photoinitiator enthalten.

Erfindungsgemäß sind jedoch solche Schlicker bevorzugt, die nicht reaktives Bindemittel enthalten, d.h. Bindemittel, das nicht radikalisch polymerisierbar ist. Im Gegensatz zu reaktiven Bindemitteln bilden nicht reaktive Bindemittel bei der Härtung kein kovalentes Polymernetzwerk. Deswegen können sich die Keramik- und/oder Glaskeramikpartikel (a) relativ zueinander verschieben und so Spannungen während des Trocknens und/oder Entbinderns teilweise abbauen.

Bevorzugt sind Bindemittel, die in reiner Form bei 25°C fest sind. Während des Trocknungsprozesses erstarren derartige Bindemittel und sorgen für eine bessere Festigkeit des Grünkörpers.

Bevorzugte Bindemittel sind Cellulosederivate wie Methyl-(MC), Hydroxyethyl- (HEC), Hydroxypropylmethyl- (HPMC) und Hydroxybutylmethylcellulose (HBMC) sowie Natriumcarboxymethylcellulose (NaCMC), sowie Cellulosederivate gemäß der folgenden Formel 1:

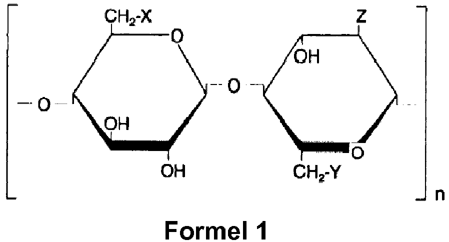

Die Variablen der Formel 1 haben die folgenden Bedeutungen:
- X: -OH, vorzugsweise -OCH₃, -OC₂H₄OC₂H₄OH, -OC₃H₆OH, -OC₄H₈OH, -OCH₂COONa
- Y: -OH, vorzugsweise -OCH₃, -OC₂H₄OH, -OCH₂COONa
- Z: -OH, -OCH₃, -OC₂H₄OC₂H₄OH, vorzugsweise -OCH₃, -OC₂H₄OC₂H₄OH.

Der Polymerisationsgrad n ergibt sich aus den unten definierten Molekulargewichten.

Bevorzugte Cellulosederivate der Formel 1 sind Verbindungen, in denen die Variablen die folgenden Bedeutungen haben:
X = Y = Z = -OCH₃
X = Z = -OC₂H₄OC₂H₄OH, Y = -OC₂H₄OH
X = -OC₃H₆OH, Y = Z = -OCH₃
X = -OC₄H₈OH, Y = Z = -OCH₃.

Andere Polysaccharide wie Agar-Agar oder Amylopektin können ebenfalls als Bindemittel verwendet werden, sind jedoch weniger bevorzugt.

Weitere bevorzugte Bindemittel sind Poly(vinylalkohol) (PVA), Poly(vinylacetat) (PVAc), Poly(vinylpyrrolidin) (PVP), Poly-(acrylsäure) (PAA), Copolymere von Acrylsäureester und Acrylsäure (AE/AA), Poly(ethylacrylat) (PEA), Poly(methacrylsäure) (PMAA), Poly(methylmethacrylat) (PMMA), Ammoniumpolyacrylat (NH4PA), Ammoniumpoly(methacrylat), Poly(acryl amide), Gelatine und Poly(ethylenglycol) (HO-(CH₂CH₂O)ₙ-OH) und Mischpolymere von Ethylenglycol und Propylenglycol, die ein so hohes Molekulargewicht und/oder einen so hohen PEG-Anteil aufweisen, dass sie bei Raumtemperatur fest sind. Der Polymerisationsgrad n ergibt sich auch hier aus den unten definierten Molekulargewichten.

Erfindungsgemäß bevorzugt sind Bindemittel, die ein Molekulargewicht von 1000 g/mol bis 500.000 g/mol, bevorzugt von 3000 g/mol bis 200.000g/mol, besonders bevorzugt von 5000 g/mol bis 100.000 g/mol haben.

Bei Poly(ethylen glycol), Poly(proyplen glycol) und Mischpolymere von Ethylenglycol und Propylenglycol wird die mittlere Molmasse Mw angegeben, die aus der nach ASTM D4274 gemessenen Hydroxyl-Zahl errechnet wird. Bei anderen Polymeren handelt es sich, wenn nicht anders angegeben, um die durch Viskosimetrie bestimmte Molmasse Mη (Viskositätsmittel) nach Ubbelohde.

Besonders bevorzugte nicht reaktive Bindemittel (b) sind Poly(vinylalcohol) (PVA) und Poly(ethylenglycol) (PEG).

Zeigen das Bindemittel und das Dispergiermedium eine starke Interaktion, wie z.B. bei polaren Bindemitteln und Wasser, kann diese Interaktion eine Verlangsamung des Trocknungsprozesses bewirken, so dass nach dem Trocknen noch Reste des Dispergiermediums vorhanden sind. Durch Art und Menge des Bindermittels kann so die Trocknungskinetik an den Prozess angepasst werden.

Andere Bindemittel, besonders die Cellulosederivate, ergeben Schlicker mit einer ausgeprägten Scherraten-abhängigen Viskosität. Dieser Effekt kann dazu genutzt werden, die Sedimentation der Partikel bei der Lagerung zu reduzieren. Während der Lagerung ist die Scherrate niedrig. Dies hat eine hohe Viskosität und damit eine langsame Sedimentation zur Folge. Während des Druckprozess bewirken die dabei auftretenden hohen Scherraten eine Verringerung der Viskosität, wodurch eine gute Verarbeitbarkeit gewährleitet wird.

Das Bindemittel wird vorzugsweise so auf das Dispergiermedium und die Keramikpartikel abgestimmt, dass eine homogene, stabile Suspension erhalten wird, d.h., dass vorzugsweise keine Flokkulation stattfindet. Die Stabilität der Suspension kann gemäß E.J.W. Verwey, J.Th.G. Overbeek: Theory of the stability of lyophobic colloids, Elsevier, New York 1948, bestimmt werden.

Das Bindemittel kann im Dispergiermedium (d) in Form kleiner Partikel dispergiert sein (sogenannte Dispersionsbinder), vorzugweise ist das Bindemittel (b) aber im Dispergiermedium (d) gelöst.

Die **Energietransformationskomponente (c)** absorbiert den Hauptteil der Energie des eingebrachten Energieimpulses, beispielsweise des einfallenden Laserstrahls, und wandelt diese in Wärme um. Der so erzeugte Wärmeimpuls wird auf die Volumenausdehnungskomponente übertragen und führt zu deren schlagartigen Ausdehnung, beispielsweise zur schlagartigen Bildung von mikroskopischen Gasbläschen durch Verdampfen der Volumenausdehnungskomponente. Hierdurch wird der Transfer des Schlickers vom Trägersubstrat auf das Empfängersubstrat induziert. Der Schlicker wird auf dem Empfängersubstrat abgeschieden.

Die Energietransformationskomponente (c) wird auf die Wellenlänge des zu absorbierenden Laserlichts abgestimmt. Als Energietransformationskomponente sind erfindungsgemäß anorganische und insbesondere organische Farbstoffe und Pigmente bevorzugt. Besonders bevorzugt sind Farbstoffe und Farbpigmente, die sich rückstandsfrei verbrennen lassen und die nach dem Entbinderungs- und Sintervorgang keine Rückstände auf dem finalen Bauteil hinterlassen.

Bevorzugt sind insbesondere Farbstoffe und Pigmente, die im Wellenlängenbereich der eingesetzten Strahlenquelle, vorzugsweise Laser, absorbieren. Hier bieten sich beispielsweise für einen Neodym-YAG Laser mit einer Wellenlänge von 1064 nm folgende Farbstoffe/Pigmente besonders an: Carbon Black, Sudan Black B (CAS 4197-25-5), Bismarck Brown Y (CAS 10114-58-6), 1-Butyl-2-[2-[3-[(1-butyl-6-chlorobenz[cd]indol-2(1H)-ylidene)-ethylidene]-2-chloro-1-cyclohexen-1-yl]ethenyl]-6-chlorobenz-[cd]indolium tetrafluoroborate (CAS 155613-98-2) oder Safranin O (CAS 477-73-6). Ganz besonders bevorzugt sind Carbon Black, Sudan Black B (CAS 4197-25-5) und Safranin O (CAS 477-73-6).

Für einen grünen Laser, z.B. mit einer Wellenlänge von 532 nm, sind folgende Farbstoffe/Pigmente bevorzugt: Carbon Black, Sudan Red 7B (Oil Violet CAS 6368-72-5), Sudan IV (CAS 85-83-6), Sudan Red G (CAS 1229-55-6), Pigment Rot 144 (CAS 5280-78-4), Safranin O (CAS 477-73-6).

Für einen blauen Laser, z.B. mit einer Wellenlänge von 405 nm, sind folgende Farbstoffe/Pigmente bevorzugt: Carbon Black, Pigment Gelb 93 (CAS 5580-57-4), Sudan Yellow 146 (CAS 4314-14-1), Disperse Yellow 7 (CAS 6300-37-4).

Die Energietransferkomponente (c) ist bevorzugt ein Bestandteil des Schlickers und ist nicht als Zusatzschicht (Absorptionsschicht) auf dem Träger vorhanden.

Die Funktion der Energietransformationskomponente kann durch Lichtstreuung, die durch Partikel der Komponente (a) und/oder der Farbmittel (e) bewirkt wird, verstärkt oder sogar übernommen werden.

Als **Dispergiermedium (d)** enthalten die erfindungsgemäßen Schlicker eine flüssige Komponente, in der die Keramik- und/oder Glaskeramikpartikel (a) dispergiert ist. Bevorzugt sind Dispergiermedium, die in reiner Form bei 25°C flüssig sind. Bevorzugte Dispergiermedium sind organische Lösungsmittel und insbesondere Wasser.

Als Dispergiermedium kann eine Mischung von verschiedenen Flüssigkeiten verwendet werden. Das Dispergiermedium enthält mindestens eine niedrigsiedende Komponente und vorzugsweise zusätzlich eine oder mehrere hochsiedende Komponenten.

Unter niedrigsiedenden Komponenten werden erfindungsgemäß Lösungsmittel mit einem Siedepunkt von weniger als 200°C (unter Normaldruck) verstanden. Geeignete Lösungsmittel sind beispielsweise, Essigsäurebutylester, Essigsäure-n-hexylester. Bevorzugte niedrigsiedende Lösungsmittel sind 1-Octanol, Propylenglycoldiacetat, Ethylenglycoldiacetat, Aceton, Methylethylketon (MEK), Isopropanol, Ethanol, Butanol, p-Xylol, Cyclohexanon, Butylacetat, Pentylacetat, Hexylacetat und besonders bevorzugt Wasser. Wasser hat den Vorteil, dass beim Verdampfen keine gesundheitsschädlichen oder explosionsgefährlichen Lösungsmitteldämpfe entstehen.

Unter hochsiedenden Komponenten werden Lösungsmittel mit einem Siedepunkt von über 200°C (unter Normaldruck) verstanden. Bevorzugte hochsiedende Lösungsmittel sind flüssige Poly(ethylen glycole) mit einem Molekulargewicht zwischen 150 bis 600 g/mol, Propylenglykol, Dipropylenglykol, Tripropylenglykol, Poly(propylglycole) mit einem Molekulargewicht von 150 bis 4000 g/mol, besonders bevorzugt mit einem Molekulargewicht von 150 bis 600 g/mol, deren Ether wie die Methyl-, Ethyl-, Propyl-, Isopropyl-, Butyl-, Hexylether, entweder als Mono- oder Diether; Phthalate, wie Dimethyl-, Diethyl-, Di-butylphthalate; Glycerin; Dimethyl-, Diethyl-, Dipropyl-, Dibutyladipate oder Glutarate; Diethylsuccinat; Acetyltri-n-butyl citrat.

Zur Kombination mit Wasser eignen sich besonders polare hochsiedende Lösungsmittel wie PEG (mittleres Molekulargewicht < 600g/mol), Glycerin und 1,2-Propandiol.

Dem Dispergiermedium (c) kommt erfindungsgemäß eine Doppelfunktion zu. Einerseits dient es als Dispergiermedium für die Komponente (a) und ggf. als Lösungsmittel für die Komponente (b), andererseits übernimmt es vorzugsweise auch die Funktion der Volumenausdehnungskomponente, wobei diese Funktion primär durch die niedrigsiedende Komponente übernommen wird.

Als Volumenausdehnungskomponente können zusätzlich oder alternativ feste, homogen dispergierte organische Substanzen eingesetzt werden, die sich einer Temperatur von 80° bis 280°C schlagartig unter Gasbildung zersetzen, beispielsweise Azobis-(isobutyronitril (AIBN). Diese dispergierten organischen Substanzen können allein oder zusammen mit der niedersiedenden Komponente des Dispergiermediums die Volumenausdehnung während des Druckprozesses bewirken.

Die niedrigsiedende Komponente des Dispergiermediums wird nach dem Tropfentransfer soweit abgedampft (getrocknet), dass sich die keramische Suspension zu einem Grünkörper verfestigt. Das Abdampfen erfolgt vorzugsweise durch einen kontrollierten Luftfluss. Wasser ist als Hauptbestandteil des Dispergiermediums bevorzugt, weil es beim Trocknen keine giftigen, brennbaren Dämpfe abgibt. Bei hydrolyseempfindlichen Partikeln, wie z.B. Lithiumdisilikatpartikeln, wird vorzugsweise ein inertes organisches Lösungsmittel verwendet, das die Partikel nicht angreift.

Neben der niedrigsiedenden Komponente kann das Dispergiermedium eine hochsiedende Komponente enthalten. Diese wird beim Trocknen nur zum Teil entfernt. Der Großteil dieser Komponente wird erst beim Entbindern entfernt, durch Verdampfen und/oder durch thermische Zersetzung.

Hochsiedende Lösungsmittel können beigemischt werden, um die Trocknungs- und oder Entbinderungskinetik zu optimieren. Mischungen aus hoch- und niedrigsiedenden Lösungsmitteln verdampfen bei unterschiedlichen Temperaturen und erlauben es so, den Prozess schonender machen. Durch die Interaktion mit dem restlichen Dispergiermedium wird beispielsweise das Trocknen verlangsamt und so das Risiko zur Ausbilden von Trocknungsrissen reduziert.

Außerdem erleichtern hochsiedende Lösungsmittel ein Gleiten der Keramikpartikel gegeneinander und ermöglichen es so, Spannungen während des Trocknens und/oder Entbinderns abzubauen. Im Gegensatz zu den Bindemitteln sind die hochsiedenden Lösungsmittel bei 25°C in reiner Form flüssig. Die hochsiedende Komponente hat damit die Funktion eines Weichmachers oder Trocknungskontrollmittels.

Neben den oben genannten Komponenten enthalten die erfindungsgemäßen Schlicker vorzugsweise zusätzlich mindestens ein **Additiv,** das aus Stabilisatoren wie Methylhydrochinon (MEHQ) und 2,6-Di-tert-butyl-p-kresol (BHT), antimikrobielle und/oder fungizide Stoffe wie Polyformaldehyd, Parabene wie Hydroxybenzoesäuremethylester oder deren Salze, Rheologiemodifizierungsmitteln wie modifizierten Fettderivaten, modifizierten Harnstoffderivate und Duftstoffen wie 2-Benzylidenheptanal (Amylzimtaldehyd), Ethyl-2-naphthylether und ätherischen Ölen ausgewählt ist.

Mit Ausnahme der Keramik- und/oder Glaskeramikpartikel (a) und der Farbmittel (e) werden alle im Schlicker befindlichen Stoffe beim Entbinderungs- und Sinterprozess entfernt. Bevorzugt sind solche Stoffe, die dabei weniger als 0,1 Gew.-% bezogen auf die Keramikphase an Rückständen bilden.

Die erfindungsgemäßen Schlicker weisen vorzugsweise folgende Zusammensetzung auf:

| **Komponente** | **bevorzugt** | **besonders bevorzugt** | **ganz besonders bevorzugt** |
|---|---|---|---|
| (Glas-)Keramikpulver (a) | 35-90% | 50-88% | 60-85% |
| Farbmittel (e) (% bezogen auf das (Glas-) Keramikpulver) | 0,00001-30% | 0,0001-25% | 0,001-20% |
| Bindemittel (b) | 0-10% | 0,2-7% | 0,5%-5% |
| Energietransformationskomponente (c) | 0,000-5% | 0,001-3% | 0,01-1,5% |
| Dispergiermedium (d) | 9-50% | 11-40% | 14-30% |
| Niedersiedende Anteile des Dispergiermediums | mind. 50% des Dispergiermediums | mind. 60% des Dispergiermediums | mind. 70% des Dispergiermediums |
| Additive | 0-15% | 0-10% | 0-5% |

Wenn nicht anders angegeben sind alle Angaben in Gewichtsprozent und beziehen sich auf das Gesamtgewicht des Schlickers.

Erfindungsgemäß bevorzugte Schlicker sind Suspensionen von Zirkonoxid in einem flüssigen Medium, die einen Gehalt an Zirkonoxid von 68 bis 88 Gew.-%, bevorzugt 70 bis 86 Gew.-% und besonders bevorzugt 75 bis 85 Gew.-% aufweisen.

Das Zirkonoxid in der Suspension hat insbesondere eine Teilchengröße von 50 bis 250 nm, bevorzugt von 60 bis 250 nm und besonders bevorzugt 80 bis 250 nm, gemessen als d₅₀-Wert, bezogen auf das Volumen aller Teilchen. Die Bestimmung der Teilchengröße erfolgt insbesondere mit dem Verfahren der statischen Laserbeugung (SLS) nach ISO 13320:2009, z.B. unter Benutzung eines Partikelanalysators LA-960 der Firma Horiba, oder der dynamischen Lichtstreuung (DLS) nach ISO 22412:2017, z.B. unter Benutzung eines Partikelmessgeräts Nano-flex der Firma Colloid Metrix.

Die Primärpartikelgröße des Zirkonoxids liegt insbesondere im Bereich von 30 bis 100 nm und sie wird üblicherweise ebenfalls mit einem wie vorstehend beschriebenen Verfahren der dynamischen Lichtstreuung (DLS) oder mittels Rasterelektronenmikroskopie bestimmt.

Bei dem Zirkonoxid handelt es sich insbesondere um Zirkonoxid auf Basis von polykristallinem tetragonalem Zirkonoxid (TZP). Bevorzugt ist Zirkonoxid, das mit Y₂O₃, La₂O₃, CeO₂, MgO und/oder CaO stabilisiert ist und insbesondere mit 2 bis 14 Mol-%, vorzugsweise mit 2 bis 10 Mol-% und besonders bevorzugt 2 bis 8 Mol.-% dieser Oxide, bezogen auf den Gehalt an Zirkonoxid, stabilisiert ist.

Das Zirkonoxid kann auch eingefärbt sein. Die gewünschte Einfärbung wird dabei insbesondere erzielt, indem das Zirkonoxid mit einem oder mehreren färbenden Elementen versetzt wird. Die Zugabe von färbenden Elementen wird bisweilen auch als Dotierung bezeichnet und sie erfolgt üblicherweise während der Herstellung des Zirkonoxidpulvers durch Cofällung und anschließende Kalzinierung. Beispiele für geeignete färbende Elemente sind Fe, Mn, Cr, Ni, Co, Pr, Ce, Eu, Gd, Nd, Yb, Tb, Er und Bi.

Bei dem Zirkonoxid in der Suspension kann es sich auch um ein Gemisch von Zirkonoxidpulvern mit unterschiedlicher Zusammensetzung handeln, die insbesondere zu einer unterschiedlichen Färbung und/oder Transluzenz bei der schließlich erzeugten dentalen Restauration führt. Mithilfe eines Gemisches aus unterschiedlich gefärbten Zirkonoxidpulvern kann somit die gewünschte Farbe einfach und in gezielter Weise eingestellt werden. In gleicher Weise kann auch die Transluzenz des erzeugten Objekts durch Verwendung eines Gemisches von Zirkonoxidpulvern unterschiedlicher Transluzenz gezielt eingestellt werden. Der Grad der Transluzenz des erzeugten Objekts kann insbesondere durch den Gehalt an Yttriumoxid der verwendeten Zirkonoxidpulver gesteuert werden.

Bei der Suspension kann es sich auch um ein Gemisch von unterschiedlichen Suspensionen zum Beispiel mit unterschiedlich gefärbtem Zirkonoxid handeln.

Das Zirkonoxid liegt als Suspension in einem flüssigen Medium vor. Dieses flüssige Medium enthält insbesondere Wasser. Es ist darüber hinaus bevorzugt, dass das flüssige Medium lediglich geringe Mengen an organischen Komponenten aufweist und es enthält daher organische Komponenten in einer Menge von insbesondere nicht mehr als 5 Gew.-%, bevorzugt nicht mehr als 3 Gew.-%, weiter bevorzugt nicht mehr als 2 Gew.-% und besonders bevorzugt nicht mehr als 1 Gew.-%, bezogen auf die Menge an Feststoff in der Suspension.

In einer weiteren bevorzugten Ausführungsform enthält das flüssige Medium organische Komponenten in einer Menge von 0,05 bis 5 Gew.-%, insbesondere 0,1 bis 3 Gew.-%, besonders bevorzugt 0,1 bis 2 Gew.-% und besonders bevorzugt 0,1 bis 1 Gew.-%, bezogen auf die Menge an Feststoff in der Suspension.

Als organische Komponenten des Schlickers kommen neben dem Bindemittel (b) insbesondere Dispergiermittel, Mittel zur Einstellung des pH-Wertes, Stabilisierungsmittel und/oder Entschäumer infrage.

Das Dispergiermittel dient dazu, die Agglomeration von suspendierten Teilchen zu größeren Teilchen zu vermeiden. Die Menge an Dispergiermittel in dem flüssigen Medium beträgt insbesondere 0,01 bis 5 Gew.-%, bevorzugt 0,1 bis 2 Gew.-% und besonders bevorzugt 0,1 bis 1 % Gew.-%, bezogen auf die Menge an Feststoff in der Suspension.

Bevorzugte Dispergiermittel für den Schlicker sind Aminoalkohole, wie Ethanolamin, Carbonsäuren, wie Maleinsäure und Citronensäure, und Carbonsäuresalze, sowie Mischungen dieser Dispergiermittel. Der Schlicker enthält vorzugsweise mindestens ein Dispergiermittel, besonders bevorzugt mindestens eine Verbindung ausgewählt aus Ethanolamin, Citronensäure und Citronensäuresalz.

Weitere bevorzugte Dispergiermittel sind Ameisensäure, Essigsäure, Propionsäure, Octansäure, Isobuttersäure, Isovaleriansäure, Pivalinsäure oder Phosphonsäuren, z.B. wie Methyl-, Ethyl-, Propyl-, Butyl- Hexyl, Octyl- oder Phenylphosphonsäure, saure Phosphorsäureester, wie z.B. Dimethyl-, Diethyl-, Dipropyl-, Dibutyl-, Dipentyl-, Dihexyl-, Dioctyl- oder Di(2-ethylhexyl)phosphat und Tetramethylamoniumhydroxid und 2 bis 8 fach ethoxylierte oder propoxilierte Catechole oder Gallate.

Das Bindemittel fördert den Zusammenhalt von Teilchen in dem Grünling. Die Menge an Bindemittel in dem flüssigen Medium beträgt insbesondere 0 bis 10 Gew.-%, bevorzugt 0,2 bis 7 Gew.- und besonders bevorzugt 0,5 bis 5 Gew.-%, bezogen auf die Menge an Feststoff in der Suspension.

Als Mittel zur Einstellung des pH-Wertes und als Stabilisierungsmittel kommen insbesondere Säuren und Basen infrage, wie Carbonsäuren, z.B. 2-(2-Methoxyethoxy)essigsäure und 2-[2-(2-Methoxyethoxy)ethoxy]essigsäure, anorganische Säuren, z.B. Salzsäure und Salpetersäure, sowie Ammoniumhydroxid und Tetramethylammoniumhydroxid. Es ist bevorzugt, dass das flüssige Medium Tetramethylammoniumhydroxid enthält.

Der Entschäumer dient der Vermeidung von Luftblasen in der Suspension. Er wird typischerweise in einer Menge von 0,001 bis 1 Gew.-%, vorzugsweise 0,001 bis 0,5 Gew.-% und besonders bevorzugt 0,001 bis 0,1 Gew.-%, bezogen auf die Menge an Feststoff in der Suspension, in dem flüssigen Medium verwendet. Beispiele für geeignete Entschäumer sind Paraffine, Siliconöle, Alkylpolysiloxane, höhere Alkoholed insbesondere Alkylpolyalkylenglykolether.

Aufgrund des geringen Anteils an organischen Komponenten können diese auch innerhalb kurzer Zeit aus dem Rohling ausgebrannt werden.

Zur Erzeugung der Suspension wird typischerweise das Zirkonoxid in Pulverform mit dem flüssigen Medium innig vermischt. Dabei können auch Gemische von zum Beispiel unterschiedlich gefärbtem Zirkonoxid verwendet werden. Bei dieser Vermischung werden üblicherweise auch vorhandene Agglomerate aufgebrochen und es kann auch ein Mahlen des eingesetzten Zirkonoxids erfolgen, um die gewünschte Teilchengröße zu erzeugen. Das Vermischen von Zirkonoxid und flüssigem Medium kann daher zum Beispiel vorteilhaft in Rührwerkskugelmühlen durchgeführt werden.

Die erfindungsgemäßen Schlicker haben vorzugsweise eine Viskosität von 0,01 Pas bis 1000 Pas. Wenn nicht anders angegeben wird die Viskosität hierin mit einem Anton Paar Rheometer mit CP50-1 Konus-Platte-Messmittel bei einer Scherrate von 100/s und bei der Verarbeitungstemperatur gemessen. Die Verarbeitungstemperatur ist wenn nicht anders angegeben 25°C.

Die Schlicker können bei Raumtemperatur (25°C) eine feste oder pastöse Konsistenz aufweisen. Dies hat den Vorteil, dass die Schlicker eine höhere Lagerstabilität haben. Sie werden in dem Fall erst beim Druckvorgang geschmolzen, entweder durch den zum Transfer verwendeten Energieimpuls oder dadurch, dass der Träger erwärmt wird, vorzugsweise auf ca. 50°C. Die Verwendung fester oder pastöser Schlicker ist besonders bei Lithiumdisilikat-Schlickern bevorzugt.

Die erfindungsgemäßen Schlicker eignen sich besonders zur Herstellung von Keramik- oder Glaskeramikformteilen, insbesondere zur Herstellung von Dentalrestauration, wie z.B. Inlays, Onlays, Veneers, Kronen, Brücken, Gerüsten, Abutments, Brackets oder Implantaten.

Die erfindungsgemäßen Schlicker werden vorzugsweise zusammen mit einem geeigneten **Stützmaterial** verdruckt. Die Stützmaterialien sollten sich in Kombination mit den verwendeten Schlickern bevorzugt inert verhalten. Die Schlicker werden zusammen, vorzugsweise sequenziell, mit dem Stützmaterial verdruckt.

Erfindungsgemäß sind Stützmaterialien bevorzugt, die keine Komponenten enthalten, die mit dem verwendeten Schlicker reagieren. Dies würde das Entfernen der Stützmaterialien aus dem Formkörper erschweren. Nach der Härtung des Werkstücks wird das Stützmaterial aus dem Formkörper entfernt. Generell sind solche Stützmaterialien bevorzugt, die ausschließlich organische Komponenten enthalten.

Bevorzugt sine Stützmaterialien, die
(α) mindestens eine Energietransformationskomponente,
(β) mindestens eine Volumenausdehnungskomponente und
(γ) mindestens ein Bindemittel enthalten.

Als Bindemittel eignen sich insbesondere bei Raumtemperatur in reiner Form feste, nichtmetallische Substanzen. Das Bindemittel bewerkstelligt primär die Stützfunktion.

Die **Energietransformationskomponente (α)** im Stützmaterial wird auf die Wellenlänge des zu absorbierenden Laserlichts abgestimmt. Als Energietransformationskomponente sind erfindungsgemäß anorganische und insbesondere organische Farbstoffe und Pigmente bevorzugt. Besonders bevorzugt sind Farbstoffe und Farbpigmente, die sich rückstandsfrei verbrennen lassen und die nach dem Entbinderungs-/Sintervorgang keine Rückstände auf dem finalen Bauteil hinterlassen.

Bevorzugt sind zudem Farbstoffe und Pigmente, die im Wellenlängenbereich der eingesetzten Strahlenquelle, vorzugsweise Laser, absorbieren. Hier bieten sich beispielsweise für einen Neodym-YAG Laser mit einer Wellenlänge von 1064 nm folgende Farbstoffe/Pigmente besonders an: Carbon Black, Sudan Black B (CAS 4197-25-5), Bismarck Brown Y (CAS 10114-58-6), 1-Butyl-2-[2-[3-[(1-butyl-6-chlorobenz[cd]indol-2(1H)-ylidene)ethyl-idene]-2-chloro-1-cyclohexen-1-yl]ethenyl]-6-chlorobenz[cd]-indolium tetrafluoroborate (CAS 155613-98-2) oder Safranin O (CAS 477-73-6) .

Für einen grünen Laser, z.B. mit einer Wellenlänge von 532 nm, sind folgende Farbstoffe/Pigmente bevorzugt: Carbon Black, Sudan Red 7B (Oil Violet CAS 6368-72-5), Sudan IV (CAS 85-83-6), Sudan Red G (CAS 1229-55-6), Pigment Rot 144 (CAS 5280-78-4), Safranin O (CAS 477-73-6).

Für einen blauen Laser, z.B. mit einer Wellenlänge von 405 nm, sind folgende Farbstoffe/Pigmente bevorzugt: Carbon Black, Pigment Gelb 93 (CAS 5580-57-4), Sudan Yellow 146 (CAS 4314-14-1), Disperse Yellow 7 (CAS 6300-37-4).

Die **Volumenausdehnungskomponente (β)** hat den Hauptzweck, einen Transfer des Druckmaterials vom Trägersubstrat auf das Empfängersubstrat zu bewirken. Damit die absorbierte Energie zu einer kontrollierten Tropfenbildung führt, soll die Volumenausdehnungskomponente durch den Wärmeimpuls in kürzester Zeit in die Gasphase überführt werden. Als Volumenausdehnungskomponente wird vorzugsweise eine Substanz mit einem Siedepunkt von 80 - 280°C und besonders bevorzugt von 95 - 200°C eingesetzt (Siedepunkte bei Normaldruck). Bevorzugte Substanzen sind 1,8-Octandiol und 1,6-Hexandiol. Besonders bevorzugt sind bei 25°C flüssige Substanzen, insbesondere Wasser und 1-Octanol. Wasser hat den Vorteil, dass beim Verdampfen keine gesundheitsschädlichen oder explosionsgefährlichen Lösungsmitteldämpfe entstehen.

Weitere bevorzugte Stoffe, die als Volumenausdehnungskomponente (β) eingesetzt werden können, sind Propylenglycoldiacetat, Ethylenglycoldiacetat, Triethyl-2-acetylcitrate, Triethylcictrat, Adibinsäuredimethylester, Adipinsäurediethylester, Triethylenglycol, Glutarsäurediethylester, Glutarsäuredimethylester, Diethylsuccinate, Essigsäurebutylester, Essigsäure-n-hexylester. Die Volumenausdehnungskomponente wird vorzugsweise so auf das verwendete Bindemittel abgestimmt, dass die Viskosität, die Oberflächenspannung und die Homogenität in den hierin definierten Bereichen liegen. Eine erfindungsgemäß geeignete Homogenität ist dann gegeben, wenn keine sichtbare Phasentrennung vorliegt. Hierzu werden polare Bindemittel wie PEG, PVA vorzugsweise mit einer polaren Volumenausdehnungskomponente wie z.B. Wasser und apolare Bindemittel wie Paraffin mit einer weniger polaren Volumenausdehnungskomponenten wie 1-Octanol kombiniert.

Alternativ können feste, homogen dispergierte organische Substanzen als Volumenausdehnungskomponente (β) eingesetzt werden, die sich bei Temperaturen von 80°-280°C schlagartig in Gase zersetzen, beispielsweise Azobis(isobutyronitril) (AIBN).

Als **Bindemittel (γ)** werden vorzugsweise Polymere, Wachse und/oder nichtionische Tenside eingesetzt.

Erfindungsgemäß bevorzugte Polymere sind Glykolpolymere, insbesondere Polyethylenglykol (PEG), Polypropylenglykol (PPG), PEG-PPG-Copolymere und PVA. Ganz besonders bevorzugt ist Polyethylenglykol (PEG) mit einem Molekulargewicht von 1500-10000g/mol. Weiter bevorzugt sind Polymere wie Polyacrylamid, Polyvinylpyrrolidon, Amylopektin, Gelatine, Cellulose, Polymere auf der Basis von Polyacrylsäure und insbesondere Copolymere von Acrylsäure oder Natriumacrylat mit Acrylamid. Diese Polymere sind polar und können Hydrogele bilden. Polare Polymere eignen sich besonders zur Kombination mit einer polaren Volumenausdehnungskomponente wie Wasser.

In der vorliegenden Erfindung ist der Begriff "Wachs" ist so zu verstehen, wie er von der Deutschen Gesellschaft für Fettwissenschaft in der DGF-Einheitsmethode MI1 (75) festgelegt wurde. Da die chemische Zusammensetzung und Herkunft verschiedener Wachse sehr unterschiedlich ist, werden Wachse über ihre mechanisch-physikalischen Eigenschaften definiert. Ein Stoff wird als Wachs bezeichnet, wenn er bei 20°C knetbar, fest bis brüchig hart, eine grobe bis feinkristalline Struktur aufweist, farblich durchscheinend bis opak, jedoch nicht glasartig ist; über 40°C ohne Zersetzung schmilzt, schon wenig oberhalb des Schmelzpunktes leicht flüssig (niedrigviskos) und nicht fadenziehend ist; eine stark temperaturabhängige Konsistenz und Löslichkeit aufweist, sowie unter leichtem Druck polierbar ist. Typischerweise gehen Wachse zwischen 40°C und 130°C in den schmelzflüssigen Zustand über; Wachse sind in der Regel wasserunlöslich. Vorzugsweise haben Wachse zur Verwendung in dem erfindungsgemäßen Stützmaterial einen Schmelzpunkt im Bereich von 40 bis kleiner 80°C, besonders bevorzugt von 45 bis 65°C.

Je nach ihrer Herkunft teilt man Wachse in drei Hauptgruppen ein, nämlich natürliche Wachse, wobei hier wiederum zwischen pflanzlichen und tierischen Wachsen, Mineralwachsen und petrochemischen Wachsen unterschieden wird; chemisch modifizierte Wachse und synthetische Wachse. Das als Bindemittel in dem erfindungsgemäßen Stützmaterial eingesetzte Wachs kann aus einer Wachsart oder auch aus Mischungen verschiedener Wachsarten bestehen.

In der vorliegenden Erfindung werden bevorzugt petrochemische Wachse, wie etwa Paraffinwachs (Hartparaffin), Petrolatum, Mikrowachs (Mikroparaffin) und deren Mischungen, besonders bevorzugt ist Paraffinwachs. Gut geeignet sind Paraffinwachse, die als Spritzgussbinder zur Fertigung von oxidkeramischen und nichtoxidkeramischen Bauteilen im Heißgießverfahren (Niederdruckspritzguss) kommerziell erhältlich sind, z.B. Paraffinwachs mit einem Schmelzpunkt ca. 54 - 56°C, einer Viskosität von 3 - 4 mPa·s bei 80°C. Häufig enthalten kommerziell erhältliche Wachse bereits Emulgatoren und/oder weitere Komponenten zur Anpassung der Rheologie.

Als Wachs können auch pflanzliche Wachse, z.B. Candelillawachs, Carnaubawachs, Japanwachs, Espartograswachs, Korkwachs, Guarumawachs, Reiskeimölwachs, Zuckerrohrwachs, Ouricurywachs, Montanwachs; tierische Wachse, z.B. Bienenwachs, Schellakwachs, Walrat, Lanolin (Wollwachs), Bürzelfett; Mineralwachse, z.B. Ceresin, Ozokerit (Erdwachs); chemisch modifizierte Wachse, z.B. Montanesterwachse, Sasolwachse, hydrierte Jojobawachse, oder synthetische Wachse, z.B. Polyalkylenwachse, Polyethylenglykolwachse, verwendet werden.

Nichtionische Tenside sind Stoffe mit grenzflächenaktiven Eigenschaften, die in wässrigen Medien keine Ionen bilden. Es handelt sich um Moleküle, die einen hydrophoben und einen hydrophilen Teil aufweisen. Durch Wahl der Länge und Art der hydrophoben und hydrophilen Teile kann die Gesamt-Hydrophobizität der Moleküle eingestellt werden.

Bevorzugt sind Stützmaterialien, die als nichtionisches Tensid (γ) ein Tensid mit einem Schmelzpunkt von 40°C bis 120°C, bevorzugt 45°C bis 80°C enthalten.

Bevorzugte nichtionische Tenside sind die Ethoxylate von Fettalkoholen, Oxoalkoholen oder Fettsäuren, Fettsäureester von Zuckern und hydrierten Zuckern, Alkylglycoside sowie Blockpolymere des Ethylen- und Propylenoxids, insbesondere kurzkettige Block-Co-Oligomere.

Besonders bevorzugt sind Fettsäuresester von hydrierten Zuckern, insbesondere solche mit der Formel R'-CO-O-Zucker, wobei R' ein verzweigter oder bevorzugt gerader Alkylrest mit 10 bis 25 Kohlenstoffatomen, vorzugsweise 12 bis 22 Kohlenstoffatomen ist. Bevorzugt sind gerade Alkylreste mit 15 bis 22 Kohlenstoffatomen. "Zucker" steht für einen hydrierten Zuckerrest, der vorzugsweise 0- bis 5-fach ethoxyliert ist. Ganz besonders bevorzugt sind Fettsäureester des Sorbitols, insbesondere Sorbitanstearate wie z.B. Sorbitanmonostearat (CAS 1338-41-6) .

Eine weitere bevorzugte Gruppe von Tensiden sind Ethoxylate von Fettsäuren, insbesondere solche mit der allgemeinen Formel R"-(CO)-(OCH₂CH₂)m-OH, in der R" ein verzweigter oder bevorzugt gerader Alkylrest mit 10 bis 25 Kohlenstoffatomen, vorzugsweise 12 bis 22 Kohlenstoffatomen ist. Besonders bevorzugt sind gerade Alkylreste mit 16 bis 22 Kohlenstoffatomen. m ist eine ganze Zahl von 0 bis 20, bevorzugt 0 bis 10 und besonders bevorzugt 0 bis 6.

Erfindungsgemäß ganz besonders bevorzugte Tenside (γ) sind Fettalkohole und Ethoxylate von Fettalkoholen, insbesondere Polyalkylenglycolether mit der allgemeinen Formel R-(OCH₂CH₂)n-OH, in der R ein Alkylrest mit 10 bis 20 Kohlenstoffatomen und n eine ganze Zahl von 0 bis 25 ist. R kann ein verzweigter oder bevorzugt gerader Alkylrest sein, wobei Alkylreste mit 12 bis 25 Kohlenstoffatomen und besonders gerade Alkylreste mit 12 bis 22 Kohlenstoffatomen bevorzugt sind. Ganz besonders bevorzugte Alkylreste sind Lauryl, Cetyl, Cetearyl und Stearyl. Die Polyalkylenglycolether sind durch Umsetzung der entsprechenden Fettalkohole mit Ethylenoxid (EO) erhältlich. Der Index n gibt die Anzahl an Ethylenoxidresten an. Polyalkylenglycolether mit 0 bis 21 (n = 2-21), insbesondere 0 bis 12 (n = 2-12) und ganz besonders 0 bis 5 (n = 2-5) Ethylenoxidresten sind bevorzugt. Beispiele für erfindungsgemäß bevorzugte Polyalkylenglycolether sind Verbindungen, in denen R ein Cetylrest (C₁₆-Rest) und n 20 und insbesondere 2 ist. Diese Verbindungen haben die INCI-Bezeichnungen Ceteth-2 und Ceteth-20. Ceteth-2 hat z.B. die Formel C₁₆H₃₃-(OCH₂CH₂)₂-OH. Weiter bevorzugt sind Verbindungen, in denen R ein Stearylrest (C₁₈-Rest) und n 2, 10, 20 oder 21 ist. Diese Verbindungen haben die INCI-Bezeichnungen Steareth-2, Steareth-10, Steareth-20 und Steareth-21. Steareth-2 hat z.B. die Formel C₁₈H₃₇-(OCH₂CH₂)₂-OH. Ganz besonders bevorzugte nichtionische Tenside sind Steareth-25 20, Steareth-10, Ceteth-20 und insbesondere Steareth-2 und Ceteth-2. Mischungen unterschiedlicher nichtionischer Tenside und insbesondere unterschiedlicher Polyalkylenglycolether können ebenfalls verwendet werden.

Bevorzugt sind Bindemittel (γ) mit einem Schmelzpunkt zwischen 40°C bis 200°C, besonders bevorzugt 50°C bis 80°C, wobei solche Bindemittel besonders bevorzugt sind, die sich beim Schmelzen nicht thermisch zersetzen. Im geschmolzenen Zustand hat das Bindemittels vorzugsweise eine Viskosität von unter 100 Pas, besonders bevorzugt unter 20 Pas und ganz besonders bevorzugt unter 5 Pas, damit es gut vom Bauteil entfernbar ist. Das Bindemittel sollte möglichst rückstandsfrei verbrennbar sein. Wichtig ist eine ausreichende Festigkeit des Stützmaterials im festen Zustand, um das Druckmaterial entsprechend unterstützen zu können.

Die erfindungsgemäßen Stützmaterialien können zur Einstellung der Oberflächenspannung und zur Einstellung der Grenzflächenspannung zwischen Stützmaterial und Träger, Stützmaterial und Empfänger und Stützmaterial und Baumaterial zusätzlich ein oder mehrere weitere Tenside enthalten. Durch die Einstellung von Oberflächen- und Grenzflächenspannung wird sichergestellt, dass sich die auf den Träger aufgebrachte Schicht des Stützmaterials nicht zusammenzieht (Bulging-Effekt), eine homogene Schicht auf dem Empfänger bildet und dass sich das Baumaterial nicht auf dem Stützmaterial zusammenzieht (Bulging-Effekt). Bevorzugte Tenside zur Einstellung der Ober- und Grenzflächenspannung sind ionische Tenside (z.B. Stearinsäure), amphotere Tenside (z.B. N,N,N-Trimethylammonioacetat) und bevorzugt die oben genannten nichtionischen Tenside, wobei hier Fettalkoholethoxylate (FAEO) und Polyalkylenglycolether besonders bevorzugt sind. Gewisse Tenside, besonders die oben definierten nichtionischen Tenside haben zusätzlich zur grenzflächenanpassenden Funktion auch eine Stützfunktion.

Die erfindungsgemäßen bevorzugten Stützmaterialen haben vorzugsweise eine Viskosität von 0,2 Pas bis 1000 Pas und eine Oberflächenspannung von 20 bis 150 mN/m, bevorzugt 30 bis 100 mN/m und besonders bevorzugt 40 bis 90 mN/m.

Wenn nicht anders angegeben wird die Viskosität der Stützmaterialien mit einem Anton Paar Rheometer mit CP50-1 Konus-Platte-Messmittel bei einer Scherrate von 100/s und bei einer Temperatur von 25°C gemessen.

Die erfindungsgemäß bevorzugten Stützmaterialien können neben den genannten Stoffen vorzugsweise ein oder mehrere **Additive** enthalten. Bevorzugte Additive sind Stabilisatoren wie Methylhydrochinon (MEHQ) und 2,6-Di-tert-butyl-p-kresol (BHT); Rheologiemodifizierungsmittel wie, Polyvinylalkohol, Hydroxyethylcellulose, Carboxymethylcellolose, Polyvinylpyrrolidon; Duftstoffe, wie 2-Benzylidenheptanal (Amylzimtaldehyd), Ethyl-2-naphthylether und ätherische Öle; und Füllstoffe. Bevorzugt sind organische Füllstoffe, die rückstandsfrei verbrennen. Als Additive kommen weiterhin antimikrobielle Stoffe wie Polyformaldehyd, Parabene wie Hydroxybenzoesäuremethylester in Betracht.

Die erfindungsgemäß bevorzugten Stützmaterialien enthalten vorzugsweise:
0,05 bis 30 Gew.-%, besonders bevorzugt 0,05 bis 20 Gew-% Energietransformationskomponente (α),
5 bis 60 Gew.-%, besonders bevorzugt 8 bis 50 Gew-% Volumenausdehnungskomponente (β),
35 bis 94, 95 Gew.-%, besonders bevorzugt 40 bis 90 Gew-% und ganz besonders bevorzugt 49 bis 90 Gew.-% Bindemittel (γ)

Alle Mengenangaben beziehen sich, wenn nicht anders angegeben, auf die Gesamtmasse des Stützmaterials.

Die Herstellung der Keramik- oder Glaskeramikformteile erfolgt durch ein LIFT-Verfahren wie hierin definiert mit den oben beschriebenen Schritten. Die Herstellung des Grünlings findet in den Schritten (1) bis (4) statt. Es wird durch schichtweises Aufbringen eines keramischen Schlickers auf ein Empfängersubstrat ein keramischer Grünling hergestellt, der in Schritt (7) entbindert wird. Hierbei wird das verwendete Bindemittel durch Erhitzen des Grünlings auf eine Temperatur von vorzugsweise 90°C bis 600°C entfernt, und es wird der sogenannte Weißling erhalten.

Die Schritte (1) und (2) des Verfahrens werden vorzugsweise bei Raumtemperatur durchgeführt. Besonders bei festen oder pastösen Schlickern kann der Druckvorgang vorteilhaft auch bei erhöhter Temperatur durchgeführt werden, beispielsweise indem der beschichtete Träger in Schritt (1) und/oder (2) erwärmt wird, vorzugsweise auf ca. 50°C.

Gemäß einer bevorzugten Ausführungsform des Verfahrens wird der Laser bzw. die Energiequelle nicht nur zum Transfer des Schlickers vom Trägersubstrat zum Empfängersubstrat eingesetzt, sondern auch dazu, die im Schlickermaterial enthaltenen organischen Komponenten ganz oder teilweise zu entfernen.

Der Weißling wird in Schritt (7) zu einem dichten keramischen Formkörper gesintert. Die Sinterung des Weißlings erfolgt im Sinterofen, vorzugsweise bei einer Temperatur für Glaskeramik von 650 bis 1100°C, bevorzugt 700 bis 900°C, für Zirkondioxid von 1100 bis 1600°C und für Aluminiumoxid von 1200 bis 1600°C, bevorzugt 1250 bis 1500°C. Das Sintern erfolgt vorzugsweise im Vakuum. Hierdurch lassen sich das Risiko der Bildung von Lufteinschlüssen und die Prozesszeit reduzieren.

Die nach dem erfindungsgemäßen Verfahren hergestellten Keramikformkörper zeichnen sich durch eine hohe Festigkeit und große Detailgenauigkeit aus. Die Biegefestigkeit nach ISO 6872 liegt bei Formkörpern aus Glaskeramik vorzugsweise über 100 MPa, insbesondere im Bereich von 150 bis 500 MPa. Formkörper aus Al₂O₃ haben eine Biegefestigkeit von vorzugsweise über 300 MPa, insbesondere von 500 bis 1000 MPa, Formkörper aus ZrO₂ von mehr als 500 MPa insbesondere von 800 bis 2500 MPa.

Die erfindungsgemäßen Schlicker ergeben eine hohe Grünkörperfestigkeit und hohe Formstabilität, Genauigkeit und Präzision nach dem Entbindern, Sintern und Reinigen. Beim Sintern kann ohne weiteres eine Dichte von > 99% der theoretischen Dichte erzielt werden. Durch das erfindungsgemäße Verfahren können insbesondere auch Schlicker mit einer deutlich höheren Viskosität verarbeitet werden als mit herkömmlichen Inkjet- oder Stereolithographieverfahren. Auf diese Weise kann der Gehalt an Keramik- und/oder Glaskeramikpartikeln im Schlicker erhöht und der Bindemittel- und Dispergiermediumgehalt entsprechend verringert werden, so dass das Entbindern erheblich beschleunigt und vereinfacht wird und die mit dem Dichtsintern verbundenen Probleme weitgehend vermieden werden.

Im Gegensatz zu herkömmlichen Stereolithographieverfahren können bei dem erfindungsgemäßen Verfahren unterschiedliche Schlicker in einer Schicht abgeschieden werden. Auf diese Weise ist zum Beispiel das Anfertigen von ortsselektiv eingefärbten dentalen Restaurationen möglich. Außerdem ist das Entbindern im Vergleich zu herkömmlichen stereolithographischen Verfahren deutlich schneller und weniger fehleranfällig. Bei dem erfindungsgemäßen Verfahren trocknen die einzelnen Schichten bereits während des Druckvorgangs zumindest teilweise, so dass die Diffusionswege für das Verdunsten der niedrigsiedenden Anteile kurz sind. Außerdem bilden sich hierbei Poren, die das Entweichen von Dispergier- und Bindemittelkomponenten sowie von Abbauprodukten beim Entbindern und Sintern erleichtern. Die bei der herkömmlichen Stereolithographie erhaltenen Grünlinge weisen demgegenüber in frühen Phasen der Entbinderung keine Poren auf, so dass das Entbindern sehr vorsichtig und langsam erfolgen muss.

Die Erfindung wird im Folgenden anhand von Ausführungsbeispielen näher erläutert.

### Ausführungsbeispiele

### Beispiel 1

### Wässriger ZrO₂-Schlicker

In 194,4 g destilliertem Wasser wurden nacheinander 3,15 g Dispergator (Dolapix CE64, Firma Zschimmer & Schwarz) und 1,5 g Tetramethylammoniumhydroxid gelöst. Die Lösung hatte einen pH-Wert von 10-10,5. Diese Lösung wurde anschließend in den Vorratsbehälter einer Rührwerkskugelmühle (MicroCer-Rührwerkskugelmühle, Firma Netzsch) gegeben, deren Mahlraum und Rotor aus Zirkonoxid bestehen. Der Mahlraum wurde mit 60 ml Zirkonoxid-Mahlperlen mit einem Durchmesser von 0,2-0,3 mm (Firma Tosoh) gefüllt. Bei einer Umdrehungsgeschwindigkeit des Rotors von 1500 U/min wurde die Lösung mit einer Schlauchpumpe (Schlauchinnendurchmesser 8 mm) kontinuierlich durch den Mahlraum gepumpt. Zu der Lösung im Vorratsbehälter wurden dann unter Rühren kontinuierlich 630 g Zirkonoxidpulver zugegeben, das mit 3 mol.-% Y₂O₃ teilstabilisiert war (Primärpartikelgröße: 40 nm; TZ-PX-245). Nach vollständiger Zugabe des Zirkonoxidpulvers wurde das erhaltene Gemisch für 45 min mit einer Rate von ca. 40 l/h kontinuierlich durch den Mahlraum und wieder in den Vorratsbehälter gepumpt. Die auf diese Weise hergestellte Suspension wurde in ein externes Gefäß zur Weiterverarbeitung überführt und mit einem Magnetrührer sehr langsam gerührt, um eingeschlossene Luftblasen zu entfernen. Es wurde zusätzlich ein Tropfen eines Entschäumers (Contraspum, Firma Zschimmer & Schwarz) zugegeben.

Im letzten Schritt wurde PEG 20.000 hinzugegeben und für weitere 1-2 Stunden homogenisiert. Es wurde ein Schlicker mit der folgenden Zusammensetzung erhalten:

| ***Anteil [Gew.-%]*** | ***Komponente*** |
|---|---|
| 78, 04% | ZrO₂, stabilisiert mit 3 mol.-% Y₂O₃ (TZ-PX-245) |
| 0,16% | Tetramethylammoniumhydroxid (TMAH) |
| 1,96% | PEG 20.000 |
| 0,39% | Dispergator (Dolapix CE64, Fa. (Zschimmer&Schwarz) |
| 19,45% | deionisiertes Wasser (Dispergiermedium) |

In den Ausführungsbeispielen handelt es sich, wenn nicht anders angegeben, bei allen Prozentangaben um Gewichtsprozent.

### Beispiel 2

### Wässriger ZrO₂-Schlicker

Auf die in Beispiel 1 beschriebene Weise wurde ein Schlicker mit der folgenden Zusammensetzung hergestellt:

| ***Anteil [Gew.-%]*** | ***Komponente*** |
|---|---|
| 80, 95% | ZrO₂, stabilisiert mit 3 mol.-% Y₂O₃ (TZ-PX-245) |
| 0,05% | 2-[2-(2-Methoxyethoxy)ethoxy]essigsäure (TODA) |
| 5% | PEG 10.000 |
| 0,1% | Safranin O (CAS 477-73-6) |
| 2% | Glycerin |
| 11,9% | deionisiertes Wasser (Dispergiermedium) |

### Beispiel 3

### Wässriger ZrO₂-Schlicker (für einen Rot-Ton)

Auf die in Beispiel 1 beschriebene Weise wurde ein Schlicker mit der folgenden Zusammensetzung hergestellt:

| ***Anteil [Gew.-%]*** | ***Komponente*** |
|---|---|
| 78,04% | ZrO₂, stabilisiert mit 9,2 Gew.-% Er₂O₃ (TZ-PX-389) |
| 0,16% | Tetramethylammoniumhydroxid (TMAH) |
| 1,96% | PEG 1.500 |
| 0,39% | Dispergator (Dolapix CE64, Fa. Zschimmer&Schwarz) |
| 19,45% | deionisiertes Wasser Dispergiermedium |

### Beispiel 4

### Wässriger ZrO₂-Schlicker (für einen Gelb-Ton)

Auf die in Beispiel 1 beschriebene Weise wurde ein Schlicker mit der folgenden Zusammensetzung hergestellt:

| ***Anteil [Gew.-%]*** | ***Komponente*** |
|---|---|
| 78,04% | ZrO₂, stabilisiert mit 5,33 Gew.-% Y₂O₃ und einem Fe₂O₃-Gehalt von 0,150 Gew.-% (TZ-PX-364) |
| 0,16% | Tetramethylammoniumhydroxid (TMAH) |
| 1,96% | PEG 6.000 |
| 0,39% | Dispergator (Dolapix CE64, Fa. Zschimmer&Schwarz) |
| 19,45% | deionisiertes Wasser (Dispergiermedium) |

Die in den Beispielen 1, 3 und 4 beschriebenen Schlicker können vor dem Verdrucken zur Einstellung des gewünschten Farbtons miteinander gemischt werden.

### Beispiel 5

### Lithiumdisilikat-Schlicker

Es wurde ein Schlicker mit der in der folgenden Tabelle angegeben Zusammensetzung hergestellt.

| ***Komponente*** | ***Anteil [Gew.-%]*** |
|---|---|
| Lithiumdisilikatglaskeramik (e.max Transpa; Firma Ivoclar Vivadent, Liechtenstein),gemahlen, D50 = 3µm | 75% |
| Solplus D540 (CAS 1000871-74-8; Lubrizol) | 1% |
| Poly(ethylen glycol) 10.000g/mol | 2% |
| Poly(ethylen glycol) 200 g/mol | 5% |
| Propylenglycoldiacetat | 10,8% |
| Methylethylketon | 6 % |
| Sudan Black B (CAS 4197-25-5) | 0,2% |

Hierzu wurden die aufgeführten Komponenten eingewogen. Das Gesamtgewicht der Schlicker betrug 100 Gramm. Dann wurden sie in einem Mischer (Speedmixer DAC 400fvz, Fa. Hauschild) fünfmal für je 2 min bei 2750 Upm homogenisiert. Zwischen den Homogenisierungsschritten wurde jeweils 5-10 Minuten gewartet, um das Material wieder abkühlen zu lassen. Der Schlicker ist bei 25°C fest, pastös und lagerstabil. Durch starke Scherung oder Erwärmen auf über 50°C wird er wieder flüssig und gut verarbeitbar.

### Beispiel 6

### Herstellung von Formkörpern mit einem LIFT-Verfahren

Die Schlicker aus den Beispielen 1 bis 4 wurden jeweils separat mit einer Doktorblade auf eine plasmabehandelte 50 µm dicke PET-Folie (Träger) aufgerakelt. Die Materialfilmdicke betrug in allen Fällen 30 µm. Der Schlicker aus Beispiel 5 wurde im erwärmten Zustand von 50°C aufgerakelt und verarbeitet.

Die Trägersubstrate wurden dann in den Arbeitsbereich des Lasers transferiert und dort innerhalb von maximal 5 Sekunden verarbeitet. Als Laser wurde ein Neodym-YAG Laser mit einer Wellenlänge von 1064 nm verwendet. Die beschichteten Träger wurden von hinten durch das Trägersubstrat hindurch mit einem Laserpuls von 100 ns mit einer Leistung von 12 mW beschossen, wobei der Laserstrahl auf einen Punkt mit einem Durchmesser von 50 µm fokussiert war. Als Empfängersubstrat wurden plasmabehandelte PET-Folien mit einer Dicke von 50 µm verwendet. Die Tropfen wurden mit einer Überlappung von 0-30pm nebeneinander auf dem Empfängersubstrat abgeschieden, während der Materialfilm kontinuierlich erneuert wurde. Der Abstand zwischen Trägersubstrat (Tropfengenerierungsort, d.h. der Stelle an der der Laser die Tropfen aus der Materialschicht herausschießt) und Empfängersubstrat betrug 300 µm.

Auf dem Empfängersubstrat wurden die Schlicker 1-5 mit einem konstanten Luftstrom über das Empfängersubstrat innerhalb 10 Sekunden so weit getrocknet, dass sie erstarren.

Das Stützmaterial wurde ortsselektiv auf das Empfängersubstrat aufgetragen. In die nicht mit Stützmaterial bedrucken Freiräume wurde der Schlicker auf die beschriebene Weise aufgebracht. In einer ersten Serie wurde jeweils einer der in den Beispielen 1 bis 5 beschriebenen Schlicker zur Herstellung eines Bauteils verwendet. In einer zweiten Versuchsserie wurden die Schlicker 1 bis 4 kombiniert und in einem Schachbrettmuster nebeneinander abgeschieden, um ein ortsselektiv eingefärbtes Bauteil zu erhalten. Die Verfestigung der Schlicker erfolgte durch Trocknen. Es wurden jeweils 5 Schichten Stützmaterial und 5 Schichten Baumaterial abgeschieden, dann wurde das Bauteil zur Anpassung der Schichthöhe mit einer Hartmetallfräse mit Materialabsaugung geglättet. Nach dem Glätten wurden weitere Schichten aufgebracht und erneut geglättet. Der Vorgang wurde so lange wiederholt, bis das Objekt fertig gedruckt war.

Das verwendete Stützmaterial hatte die folgende Zusammensetzung:

| ***Komponente*** | ***Anteil*** |
|---|---|
| Deionisiertes Wasser | 49,80 Gew.-% |
| Polyethylenglycol 2.000 g/mol | 50 Gew.-% |
| Safranin 0 (CAS 477-73-6) | 0,20 Gew.-% |

Nach Abschluss des Druckvorgangs wurden die gedruckten Objekte, die vom Stützmaterial vollständig umgeben waren, in einen Sinterofen gegeben, dort mit einer Heizrate von +l°C/min von Raumtemperatur auf 500°C erwärmt. Während des Aufheizens schmolz das Stützmaterial und floss aus den gedruckten Objekten heraus. Die Objekte konnten sich anschließend ohne Wiederstand beim Entbindern und Sintern zuerst leicht ausdehnen und dann zusammenziehen. Anfällig anhaftende Stützmaterialreste und das Empfängersubstrat (PET-Folie) zersetzten sich im weiteren Aufheizprozess rückstandsfrei. Danach waren die Grünkörper vollständig entbindert und konnten dichtgesintert werden. Objekte aus den Schlickern der Beispiele 1-4 (ZrO₂) wurden mit einer Heizrate von +10°C/min auf 1500°C erhitzt und dann bei 1500°C für 1 Stunde gesintert.

Objekte aus dem Schlicker gemäß Beispiel 5 (Lithiumdisilikat Schlicker) wurden ab 500°C bei einem Vakuum mit 10°C/min auf 700°C erwärmt, dort für 30 min gehalten, dann mit +10°C/min bis 850°C erwärmt, dort für 10 Minute gehalten, dann mit - 10°C/min auf 700°C kontrolliert und danach unkontrolliert weiter abgekühlt.

Nach Abkühlen auf Raumtemperatur erhielt man 3D Druckteile aus Keramik. Die Versuche zeigen, dass sich das erfindungsgemäße Verfahren und die erfindungsgemäßen Schlicker besonders zur Herstellung dentaler Restaurationen und von ortsselektiv eingefärbten Keramikformteilen eignet. Die zahnkronenförmigen, keramischen Bauteile wiesen keine Entbinderungsrisse auf, waren optische homogen und hatten eine Dichte von mehr als 99,5% der theoretischen Dichte. Die Dichte wurde nach Archimedes-Methode gemessen.

## Patentansprüche

1. Schlicker zur Herstellung von Keramik- oder Glaskeramikformteilen für den Energieimpuls induzierten Transferdruck (LIFT), der
(a) Keramik- und/oder Glaskeramikpartikel,
(b) vorzugsweise mindestens ein Bindemittel,
(c) mindestens eine Energietransformationskomponente und
(d) mindestens ein Dispergiermedium enthält.

2. Schlicker nach Anspruch 1, der als Komponente (a) Keramikpartikel auf der Basis von ZrO₂, Al₂O₃ oder ZrO₂-Al₂O₃ enthält, oder auf der Basis von ZrO₂, Al₂O₃, ZrO₂-Al₂O₃, das jeweils mit CaO, Y₂O₃, La₂O₃ CeO₂ und/oder MgO stabilisiert ist, auf der Basis von ZrO₂-Spinell, ZrO₂-Al₂O₃-Spinell, eines Spinells des Typs AB₂O₄, AB₁₂O₁₉, AB₁₁O₁₈, AB₂O₄, wobei A bevorzugt ein Alkali- oder Erdalkalimetallion und B ein Übergangsmetallion ist, das eine höhere Oxidationsstufe als A aufweist.

3. Schlicker nach Anspruch 2, bei dem die Keramikpartikel zusätzlich Er₂O₃, Fe₂O₃, Co₃O₄, MnO₂, NiO₂, Cr₂O₃, Pr₂O₃, Tb₂O₃ und/oder Bi₂O₃ als farbgebende Komponente enthalten.

4. Schlicker nach Anspruch 1, der als Komponente (a) Glaskeramikpartikel auf der Basis von Leucit-, Apatit- und/oder Lithiumdisilikatglaskeramik enthält.

5. Schlicker nach einem der Ansprüche 1 bis 4, der als Bindemittel (b) ein Bindemittel enthält, das nicht radikalisch polymerisierbar ist und das in reiner Form bei 25°C fest ist.

6. Schlicker nach Anspruch 5, der als Bindemittel (b) ein Cellulosederivat, vorzugsweise Methyl-, Hydroxyethyl-, Hydroxypropylmethyl-, Hydroxybutylmethylcellulose und/oder Natriumcarboxymethylcellulose, Poly(vinylalkohol) (PVA), Poly(vinylacetat) (PVAc), Poly(vinylpyrrolidin) (PVP), Poly(acrylsäure) (PAA), ein Copolymer von Acrylsäureester und Acrylsäure (AE/AA), Poly(ethylacrylat) (PEA), Poly-(methacrylsäure) (PMAA), Poly(methylmethacrylat) (PMMA), Ammoniumpolyacrylat (NH4PA), Ammoniumpoly(methacrylat) oder Poly(ethylenglycol) (HO-(CH₂CH₂O)-OH) enthält.

7. Schlicker nach einem der Ansprüche 1 bis 6, der als Energietransformationskomponente (c) einen anorganischen oder organischen Farbstoff oder ein anorganisches oder organisches Pigment enthält.

8. Schlicker nach einem der Ansprüche 1 bis 7, der als Dispergiermedium (d) ein Lösungsmittel mit einem Siedepunkt von weniger als 200°C (unter Normaldruck) enthält, vorzugsweise Triethyl-2-acetylcitrat, Essigsäurebutylester, Essigsäure-n-hexylester, besonders bevorzugt 1-Octanol, Propylenglycoldiacetat, Ethylenglycoldiacetat, Aceton, Methylethylketon (MEK), Isopropanol, Ethanol, Butanol, p-Xylol, Cyclohexanon, Butylacetat, Hexylacetat und besonders bevorzugt Wasser.

9. Schlicker nach Anspruch 8, bei dem das Dispergiermedium zusätzlich ein Lösungsmittel mit einem Siedepunkt von über 200°C (unter Normaldruck) enthält, vorzugsweise PEG (mittleres Molekulargewicht < 600 g/mol), Glycerin und 1,2-Propandiol.

10. Schlicker nach einem der Ansprüche 1 bis 9, der zusätzlich mindestens ein Additiv enthält, das aus Stabilisatoren, Bioziden, Parabenen, Rheologiemodifizierungsmitteln und/oder Duftstoffen ausgewählt ist.

11. Schlicker nach einem der Ansprüche 1 bis 10, der
- 35 bis 90 Gew.-%, vorzugsweise 50 bis 88 Gew.-%, besonders bevorzugt 60 bis 85 Gew.-% Keramik- und/oder Glaskeramikpartikel (a),
- 0,0 bis 10 Gew.-%, vorzugsweise 0,2 bis 7 Gew.-%, besonders bevorzugt 0,5 bis 5 Gew.-% Bindemittel (b);
- 0,000 bis 2 Gew.-%, vorzugsweise 0,001 bis 1 Gew.-%, besonders bevorzugt 0,01 bis 0,5 Gew.-% Energietransformationskomponente (c);
- 9 bis 50 Gew.-%, vorzugsweise 11 bis 40 Gew.-%, besonders bevorzugt 14 bis 30 Gew.-% Dispergiermedium (d) enthält,
jeweils bezogen auf die Gesamtmasse des Schlickers.

12. Verwendung eines Schlickers gemäß einem der Ansprüche 1 bis 11 zur Herstellung von Keramik- oder Glaskeramikformteilen, vorzugsweise einer Dentalrestauration, wie einem Inlay, Onlay, Veneer, einer Krone, Bracket, Brücke oder eines Gerüsts, Abutments oder Implantats durch Energieimpuls induzierten Transferdruck (LIFT).

13. Verfahren zur generativen Herstellung eines Keramik- oder Glaskeramikformteils, das die folgenden Schritte umfasst:
(1) flächiges Aufbringen eines Schlickers auf einen Träger in definierter Schichtdicke, vorzugsweise in einer Schichtdicke von 3 - 300 µm, besonders bevorzugt 10 - 100 µm,
(2) Transfer eines Anteils des Schlickers vom Trägersubstrat (Donor) auf ein Empfängersubstrat (Akzeptor) durch den lokalen, ortsselektiven Eintrag eines Energieimpulses, vorzugsweise eines Laserimpulses,
(3) Verfestigen des Schlickers auf dem Empfängersubstrat, vorzugsweise durch Trocknen, Strahlenhärtung oder Ändern des Aggregatszustandes,
(4) Wiederholen der Schritte (1)-(3), um einen Grünling zu erhalten,
(5) ggf. Entfernen des Stützmaterials aus dem Grünling und optionales Reinigung des Grünlings,
(6) optionales Nachvergüten des Grünlings durch weiteres Härten, vorzugsweise durch Trocknung, Strahlung, Wärme oder eine Kombination davon,
(7) Wärmebehandlung des Grünlings zum Entfernen der nicht-keramischen Inhaltsstoffe (Entbinderung), um einen Weißlings zu erhalten, und Sintern des Weißlings zu einem keramischen Objekt,
**dadurch gekennzeichnet, dass** man mindestens einen Schlicker gemäß einem der Ansprüche 1 bis 11 einsetzt.

14. Verfahren nach Anspruch 13, bei dem man den Schlicker im Anschluss an Schritt (3) glättet, vorzugsweise mit einer Walze, Klinge, Fräse und/oder einem Abstreifer.

15. Verfahren nach Anspruch 13 oder 14, bei dem man in Schritt (2) zusätzlich ein Stützmaterial auf das Empfängersubstrat aufbringt.

16. Verfahren nach einem der Ansprüche 13 bis 15, bei dem man als Träger in Schritt (1) und/oder als Empfängersubstrat in Schritt (2) eine Polymerfolie, einen Glasträger, einen Träger aus einem nicht-metallischen, anorganischen, nichtporösen Werkstoff, einen metallischen Träger oder einen keramischen Träger einsetzt.

17. Verfahren nach einem der Ansprüche 13 bis 16, bei dem der Energieeintrag in Schritt (2) über die dem Schlicker abgewandte Seite des Trägersubstrats erfolgt.
